# EUROPÄISCHE PATENTSCHRIFT

(11) **EP 2 016 960 B1**
(45) Veröffentlichungstag und Bekanntmachung des Hinweises auf die Patenterteilung: **23.03.2016**
(21) Anmeldenummer: 08158973.1
(22) Anmeldetag: 25.06.2008
(51) Int. Cl.: A61L 31/16, A61L 31/10, A61F 2/91, A61F 2/915

(54) **MEDIKAMENTENDEPOTS FÜR MEDIZINISCHE IMPLANTATE**
MEDICAMENT RESERVOIRS FOR MEDICAL IMPLANTS
RÉSERVOIRS DE MÉDICAMENTS POUR IMPLANTS MÉDICAUX

(30) Priorität: 20.07.2007 DE 102007034041
(43) Veröffentlichungstag der Anmeldung: 21.01.2009
(73) Patentinhaber: Biotronik VI Patent AG, 6341 Baar (CH)
(72) Erfinder: Klocke, Björn, 8006, Zürich (CH); Diener, Tobias, 91058, Erlangen (DE); Fringes, Matthias, 91522, Ansbach (DE); Harder, Claus, 91080, Uttenreuth (DE)
(74) Vertreter: Randoll, Sören

(56) Entgegenhaltungen:
- EP-A1- 1 389 471
- WO-A2-2005/079335
- US-A1- 2005 220 843
- US-A1- 2006 241 742

## Beschreibung

Die vorliegende Erfindung betrifft ein Verfahren zur Herstellung eines Wirkstoffdepots, ein Verfahren zur Herstellung eines wirkstoffbeschichteten, endovaskulären implantierbaren Körpers, einen wirkstoffbeladenen, endovaskulären implantierbaren Körper, hergestellt nach einem erfindungsgemäßen Verfahren sowie ein Kit umfassend oder bestehend aus einem oder mehreren Wirkstoffdepots, hergestellt nach einem erfindungsgemäßen Verfahren und ein oder mehrere endovaskuläre implantierbare Körper.

Endovaskuläre implantierbare Körper werden in der Medizintechnik unter anderem dafür verwendet, Gefäßstrukturen zu unterstützen. Insbesondere um koronare Herzerkrankungen, insbesondere akute Myokardinfarkte, zu behandeln, werden endovaskuläre Prothesen bzw. Implantate, insbesondere endovaskuläre Stents, verwendet. Solche Körper sind ebenfalls bekannt für die Behandlung von Aneurismen. Stents weisen grundsätzlich eine Tragstruktur auf, die geeignet ist, die Wand eines Gefäßes in geeigneter Weise abzustützen, und so das Gefäß zu weiten bzw. ein Aneurisma zu überbrücken.

Dazu werden Stents in einem komprimierten Zustand in das Gefäß eingeführt und dann an dem zu behandelnden Ort aufgeweitet und gegen die Gefäßwand gedrückt. Dieses Aufweiten kann beispielsweise mit Hilfe eines Ballonkatheters erfolgen. Alternativ sind auch selbstexpandierende Stents bekannt. Diese sind beispielsweise aus einem superelastischen Metall, wie Nitinol, aufgebaut.

Mit dem Aufweiten des Blutgefäßes entstehen allerdings kleinste Verletzungen und Einrisse (Dissektionen) in der Gefäßwand, die zwar häufig problemlos verheilen, jedoch in etwa einem Drittel der Fälle durch das ausgelöste Zellwachstum zu Wucherungen (Proliferation) und letztendlich zu einer erneuten Gefäßverengung (Restenose) führen können. Die Aufweitung des Gefäßes durch endovaskuläre Stents beseitigt zudem nicht die Ursachen der zugrunde liegenden Stenose, also die molekularpathologischen Veränderungen in der Gefäßwand. Eine Ursache der Restenose ist des Weiteren die übermäßige Elastizität des durch den Stent gedehnten Blutgefäßes. Üblicherweise zieht sich nach Entfernen des Ballons das gedehnte Blutgefäß übermäßig zusammen, so dass der Gefäßquerschnitt gegenüber dem nicht gedehnten Areal des Blutgefäßes verringert wird (Obstruktion, so genanntes negatives Remodelling). Letzterer Effekt kann durch die Platzierung eines endovaskulären Implantats, in der Regel eines Stents, vermieden werden.

Zwar hat die Einführung der Stents in der interventionellen Therapie der stabilen und instabilen Angina pectoris bei koronaren Herzerkrankungen zu einer deutlichen Reduktion der Rate an Restenosen und damit zu besseren Langzeitresultaten geführt, was primär dem Lumengewinn zuzuschreiben ist, allerdings können die auftretenden kleinsten Verletzungen, welche die Proliferation induzieren können, wiederum eine Restenose auslösen. Zudem kann die Anwesenheit eines derartigen Fremdkörpers in dem Gefäßsystem eine Kaskade von zellulären molekularen Prozessen initiieren, die zu einem allmählichen Zuwachsen des Gefäßes (insbesondere auch Thrombenbildung) in dem Bereich, in dem das Implantat, insbesondere der Stent implantiert ist, führen können.

Seit einigen Jahren versucht man deshalb die Restenosegefahr bei der Implantation von Stents durch die Verwendung von mit Wirkstoff beschichteten Stents weiter zu vermindern (Local Drug Delivery (LDD); Drug Eluting Stents (DES)). Aber auch in nicht koronarbezogenen Erkrankungen kommen Wirkstoffträger zum Einsatz, die in Gefäße implantiert werden (Drug Reservoirs für nicht koronare Anwendungen (Krebstherapie etc.)).

Die Träger derartiger wirkstoffhaltiger Beschichtungssysteme bestehen regelmäßig aus einem biokompatiblen Material, welches entweder natürlichen Ursprungs ist oder auf synthetischem Wege gewonnen werden kann. Zur Aufbringung der Beschichtungssysteme auf den Stent sind zahlreiche Verfahren entwickelt worden, wie beispielsweise Rotationszerstäubungsverfahren, Tauchverfahren und Sprühverfahren. Das Beschichtungssystem bedeckt zumindest bereichsweise die Oberfläche des Stents, wobei eine Freisetzung der pharmakologischen Wirkstoffe im menschlichen bzw. tierischen Körper durch allmähliche Degradation des Trägers und/oder Diffusion in das umgebende Gewebe erfolgt.

Unter einer wirkstoffhaltigen Beschichtung von endovaskulären Stents ist üblicherweise eine flächige Beschichtung zu verstehen. Zum Teil können aber auch die Beschichtungen darin bestehen, dass die auftretenden Löcher bzw. Kavitäten der Stentgeometrie gefüllt sind oder einseitige oder punktförmige Beschichtungen auf der Tragstruktur der Stents bestehen. Solche Beschichtungen sind allerdings technologisch sehr anspruchsvoll als auch zeit- sowie kostenintensiv.

Man hat nun festgestellt, dass jedoch nicht alle Beschichtungsmaterialien mit/und ohne inkorporierte(m) Wirkstoff(en) direkt auf den implantierbaren Körper, gemäß der üblicherweise verwendeten Verfahren, beschichtet werden können.

Im Allgemeinen ist bei den meisten Beschichtungsverfahren ein vorheriges Lösen der Polymermatrix, vor der Beschichtung, notwendig. Um jedoch die notwendige Lösemittelfreiheit des "Drug Eluting" Implantates sicher zu stellen, müssen teilweise aufwendige Verfahrensschritte zur Extraktion desselben Lösungsmittels, die dem Beschichtungsschritt nachgeschaltet sind, vorgenommen werden.

Aufgrund unterschiedlicher physiko-chemischer Eigenschaften von Substratoberfläche und Beschichtungsmaterial (hydrophobe, hydrophile Eigenschaften) können ggf. die gewünschten Oberflächeneigenschaften des herzustellenden Implantates nicht erzielt werden. Um diese Eigenschaften dennoch zu erreichen, sind zum Teil aufwändige Verfahrensschritte zur Vorbehandlung der jeweiligen Oberfläche notwendig, was zu einem zusätzlichen Fertigungsschritt auch ggf. Folgen für die Biokompatibilität haben könnte.

Auch können wirkstoffbeladene Polymerschichten bei hoher Wirkstoffbeladung insbesondere zu mechanischen Problemen der Schicht bei der Dilatation des Stents führen.

EP 1 389 471 und US 2005/0220843 offenbaren einen Stent mit einer zumindest abschnittweisen polymeren Beschichtung, welche einen oder mehrere Wirkstoffe enthalten kann.

WO 2005/079335 offenbart Wirkstoff-enthaltende Fäden bestehend aus einem polymeren Material, welche mittels Verschlaufung um die Streben eines Stents gewickelt und auf diese Weise verbunden werden.

Bei den bekannten Beschichtungsformen von Stents ist man üblicherweise auf eine Wirkstoff-Dosis-Kombination beschränkt, da es sehr schwierig ist, Beschichtungen zu realisieren, welche unterschiedliche Elutionsraten eines Wirkstoffes oder Elutionen mehrerer Wirkstoffe (multiple-drug-release, insbesondere dual-drug-release, tripple-drug-release etc.), insbesondere in unterschiedlichen Elutionsraten, besitzen.

Eine Aufgabe der vorliegenden Erfindung besteht deshalb darin, ein Wirkstoffdepot bereitzustellen, das an einen endovaskulär implantierbaren Körper, unabhängig von dessen Material, gebunden werden kann. Eine weitere Aufgabe der vorliegenden Erfindung besteht darin, dass ein endovaskulär implantierbarer Körper bereitgestellt wird, der ein oder mehrere Wirkstoffdepots mit ein oder mehreren Wirkstoffen umfasst, wobei der oder die Wirkstoffe gezielt in Bezug auf die Elutionszeit und -konzentration eingestellt werden können. Eine weitere Aufgabe besteht darin, unerwünschte Implantatnebenwirkungen, z.B. Erhöhung des Restenose- und/oder Thrombosebildungsrisikos zu vermindern.

Die erfindungsgemäßen Aufgaben werden gelöst durch ein Verfahren zur Herstellung eines Wirkstoffdepots, das zur mechanischen Verbindung mit einer Oberfläche eines endovaskulär implantierbaren Körpers ausgebildet ist, umfassend oder bestehend aus folgenden Schritten:
a) Bereitstellung eines oder mehrerer Polymere,
b) Bereitstellung eines oder mehrerer Wirkstoffe und
c) Herstellung eines Wirkstoffdepots aus dem oder den Polymeren und dem oder den Wirkstoffen, wobei das Wirkstoffdepot so ausgebildet ist, dass es mittels Krafteinwirkung mit der Oberfläche des Körpers mechanisch verbindbar ist, in der Weise, dass das Wirkstoffdepots an die Oberfläche des Körpers angeklippt wird.

Ein weiterer Gegenstand der vorliegenden Erfindung betrifft ein Verfahren zur Herstellung eines wirkstoffbeschichteten endovaskulär implantierbaren Körpers, umfassend oder bestehend aus folgenden Schritten:
a) Bereitstellung eines endovaskulär implantierbaren Körpers,
b) Bereitstellung eines oder mehrerer unterschiedlicher Wirkstoffdepots, das oder die erfindungsgemäß hergestellt sind und
c) mechanische Verbindung des oder eines der Wirkstoffdepots aus Schritt b) mit dem Körper aus Schritt a), in der Weise, dass das Wirkstoffdepots an die Oberfläche des Körpers angeklippt wird.

Ein weiterer Gegenstand der vorliegenden Erfindung betrifft einen wirkstoffbeladenen, endovaskulär implantierbaren Körper, hergestellt nach einem erfindungsgemäßen Verfahren.

Ein weiterer Gegenstand der vorliegenden Erfindung betrifft ein Kit umfassend oder bestehend aus:
a) einem oder mehreren Wirkstoffdepots hergestellt nach einem erfindungsgemäßen Verfahren und
b) einem oder mehreren endovaskulär implantierbaren Körpern.

Ein weiterer Gegenstand der vorliegenden Erfindung betrifft eine Verwendung eines oder mehrerer erfindungsgemäß hergestellter Wirkstoffdepots zur Herstellung eines wirkstoffbeladenen, endovaskulär implantierbaren Körpers, bevorzugt Stents, zu Prophylaxe oder Behandlung einer Stenose, eines Aneurisma oder eines Tumorgewebes in einem menschlichen oder tierischen Körper.

Ein weiterer Gegenstand der vorliegenden Erfindung betrifft ein Verfahren zur Prophylaxe oder Behandlung einer Stenose, eines Aneurismas oder eines Tumorgewebes in einem menschlichen oder tierischen Körper umfassend oder bestehend aus folgenden Schritten:
a) Bereitstellung eines endovaskulär implantierbaren Körpers, bevorzugt eines Stents,
b) Bereitstellung eines oder mehrerer unterschiedlicher Wirkstoffdepots, das oder die erfindungsgemäß hergestellt sind,
c) mechanische Verbindung des oder eines der Wirkstoffdepots aus Schritt b) mit dem Körper aus Schritt a), in der Weise, dass das Wirkstoffdepots an die Oberfläche des Körpers angeklippt wird, und
d) Implantation des in Schritt c) hergestellten wirkstoffbeladenen Körpers in ein Blutgefäß, dass Blut zum Tumorgewebe führt.

Der vorliegenden Erfindung liegt die Erkenntnis zugrunde, dass ein erfindungsgemäßes Wirkstoffdepot, das separat von dem eigentlichen endovaskulär implantierbaren Körper gefertigt wird, zum einen in Bezug auf die Elutionscharakteristik des oder der inkorporierten Wirkstoffe technologisch einfacher optimiert werden kann und zum anderen unabhängig von dem verwendeten Material des implantierbaren Körpers, bevorzugt Stents, direkt mit diesem mechanisch verbunden werden kann. Somit können die erfindungsgemäß hergestellten Wirkstoffdepots, die mit den endovaskulär implantierbaren Körpern, vorzugsweise formschlüssig, mechanisch verbunden werden, in verbesserter Weise gegenüber dem Stand der Technik die gestellten Aufgaben lösen.

Vorteile eines erfindungsgemäß hergestellten implantierbaren Körpers mit erfindungsgemäßen Wirkstoffdepots bestehen demzufolge insbesondere darin, dass infolge der technologischen Trennung der Herstellung des implantierbaren Körpers und des Wirkstoffdepots eine direkte Wirkstoffbeladung des implantierbaren Körpers unabhängig von dem Material des implantierbaren Körpers ermöglicht wird.

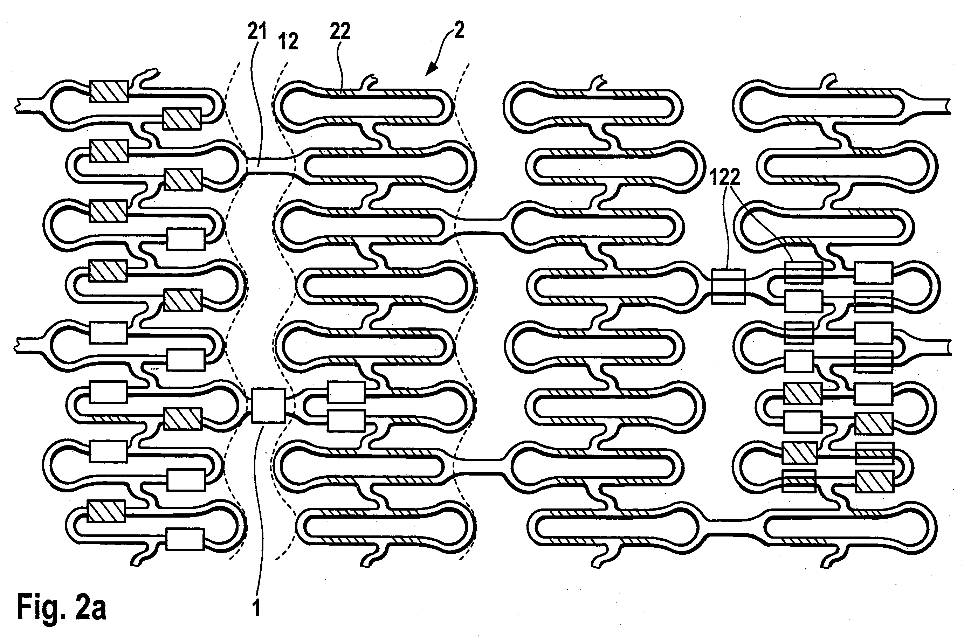

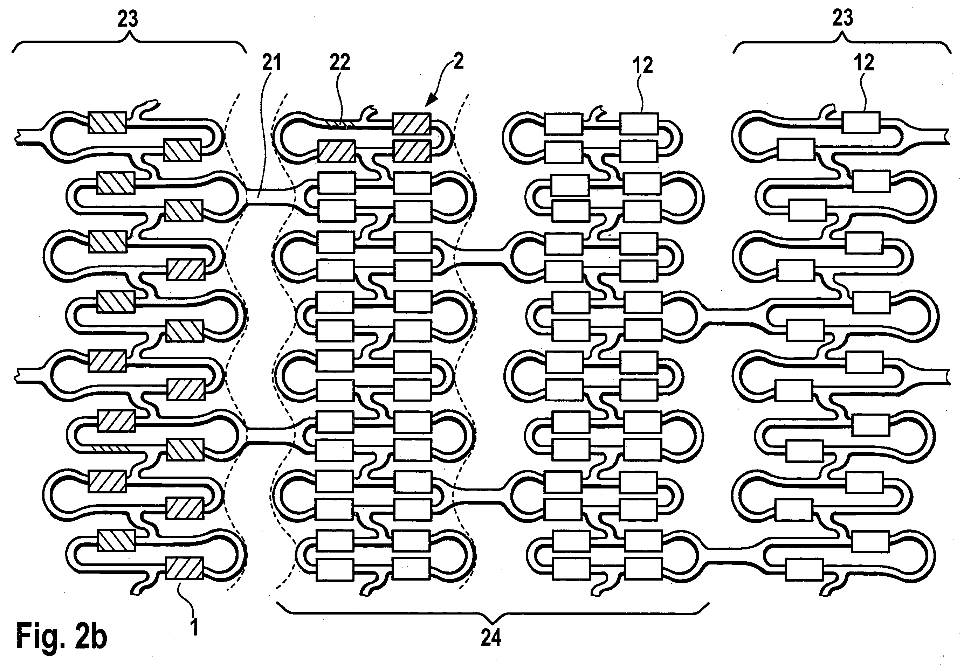

Ein weiterer Vorteil besteht darin, dass eine Beladung eines erfindungsgemäß hergestellten implantierbaren Körpers mit einem oder mehreren erfindungsgemäßen Wirkstoffdepots eine flexible, für die jeweilige Erkrankung maßgeschneiderte Wirkstofftherapie des implantierbaren Körpers ermöglicht. Eine Beladung eines Implantates mit einem oder mehreren erfindungsgemäßen Wirkstoffdepots ist technologisch einfach und dementsprechend können auch bereits vorhandene endovaskulär implantierbare Körper, seien sie wirkstoffbeschichtet oder nicht, mittels der erfindungsgemäßen Wirkstoffdepots (nach)beladen werden. Es können auch insbesondere in unterschiedlichen räumlichen Bereichen des endovaskulär implantierbaren Körpers, bevorzugt Stents, unterschiedlich viele Wirkstoffdepots beladen werden. Erfindungsgemäße Beispiele hierfür werden in Figuren 2a und 2b dargestellt. Solche erfindungsgemäßen Ausgestaltungen können insbesondere dann von Vorteil sein, wenn eine individuell auf einen Patienten maßgeschneiderte Wirkstofftherapie erforderlich ist. In diesem Fall kann sogar der behandelnde Arzt selbst die Beladung eines endovaskulär implantierbaren Körpers, bevorzugt Stents, maßgeschneidert vornehmen.

Ein weiterer Vorteil der erfindungsgemäßen Gegenstände besteht darin, dass nicht nur ein, sondern auch mehrere Wirkstoffe in dem Wirkstoffdepot insbesondere in (unterschiedlichen) Dosierungen vorliegen können, die - aufgrund der Limitierung durch beispielsweise die Schichtdicke - mit herkömmlichen Beschichtungen implantierbarer Körper nicht erreicht werden können.

Insbesondere können hier zeitlich abgestufte Elutionskurven eines oder mehrerer Wirkstoffe in dem Wirkstoffdepot verwirklicht werden, beispielsweise eine kontinuierliche Erhöhung eines oder mehrerer Wirkstoffe, um den Körper, in den das Implantat mit Wirkstoffdepot implantiert wird, schrittweise an das oder die Wirkstoffe zu gewöhnen.

Bei üblichen wirkstoffbeschichteten Stents führen Beladungen von über 70 % Wirkstoff zu schlechten mechanischen Eigenschaften, insbesondere zu einem Aufbrechen der Wirkstoffschicht, sowie zu oftmals sehr kurzen Elutionskinetiken, die nicht immer erwünscht sind.

Aufgrund der üblichen Schichtdicken von 50 bis 100 µm können Stentgeometrien entstehen, die ein Maß an Volumen des Gefäßlumens einnehmen, dass bei der Implantation des Stents (Deliverability) Probleme beim Passieren von Stenosen oder anderen Engstellen entstehen können (großes "Crossing Profile").

Im Gegensatz zu den üblichen Beschichtungsmethoden erlaubt die Verwendung erfindungsgemäßer Wirkstoffdepots, die an einen zu implantierenden Körper, bevorzugt Stent, angeklippt werden, zum einen die Ausnutzung des Platzes zwischen den Struts bzw. Stegen der zu implantierenden Stents oder das Anklippen der erfindungsgemäßen Wirkstoffdepots endständig an die zu implantierenden Körper, insbesondere Stents. Gerade bei dem endständigen Anklippen können Wirkstoffdepots verwendet werden, die eine hohe Konzentration an ein oder mehreren Wirkstoffen umfassen kann.

Hohe Wirkstoffkonzentrationen sind insbesondere von Vorteil für Wirkstoffe aus der Gruppe der Limus-Wirkstoffe zur Behandlung der Restenose, insbesondere umfassend Sirolimus (Rapamycin), Zotarolimus (Abt-578), Tacrolimus (Fk506), Everolimus, Biolimus, insbesondere Biolimus A9, Paclitaxel (Taxol), Pimecrolimus, Lipidregulatoren, vorzugsweise Fibrate, Immunsuppressiva, Vasodilatatoren, vorzugsweise Sartane, Calciumkanalblocker, Calcineurininhibitoren, vorzugsweise Tacrolimus, Antiphlogistika, vorzugsweise Kortison und Diclofenac, Antiinflammatorika, vorzugsweise Imidazole, Antiallergika, Oligonucleotide, vorzugsweise Decoy-Oligodesoxynukleotid (dODN), Estrogene, vorzugsweise Genistein, Endothelbildner, vorzugsweise Fibrin, Steroide, Proteine/Peptide, Proliferationshemmer, Analgetika, Antirheumatika etc.

Höhere Wirkstoffkonzentrationen können ebenfalls für lokale Krebstherapien wünschenswert sein, so dass der oder die Wirkstoffe aus der Gruppe der Zytostatika, sofern das Implantat in ein dem Tumorgewebe zuführendes Gefäß, bevorzugt nahe dem Tumorgewebe, platziert wird, dort direkt in das Blutgefäß freigesetzt werden und in hoher Konzentration direkt mit dem Blutstrom dem Tumorgewebe zugeführt werden können. Es besteht hierbei die Möglichkeit, dass Nebenwirkungen, die mit den verwendeten Zytostatika assoziiert sind, verringert werden können.

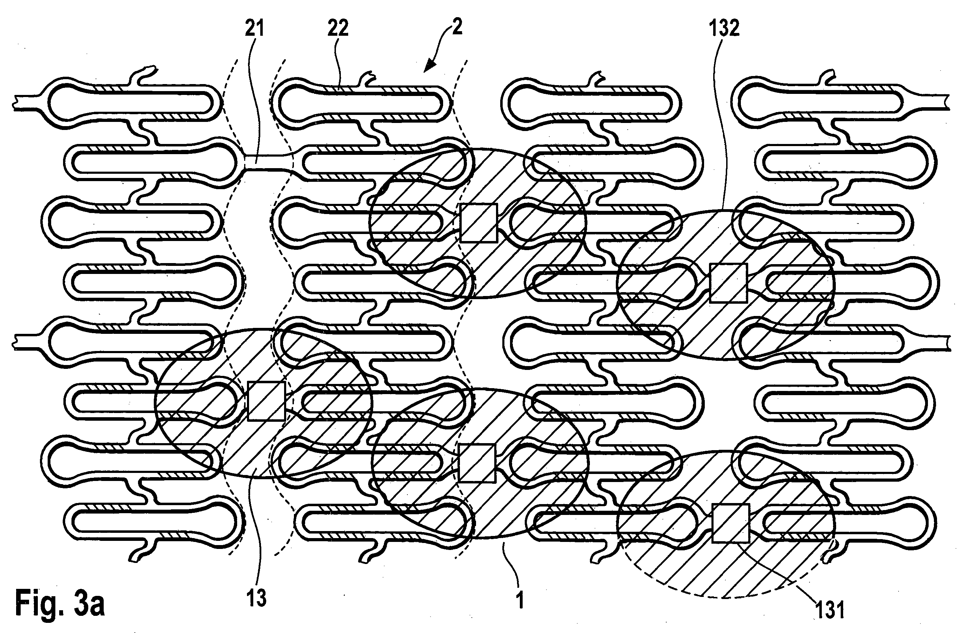

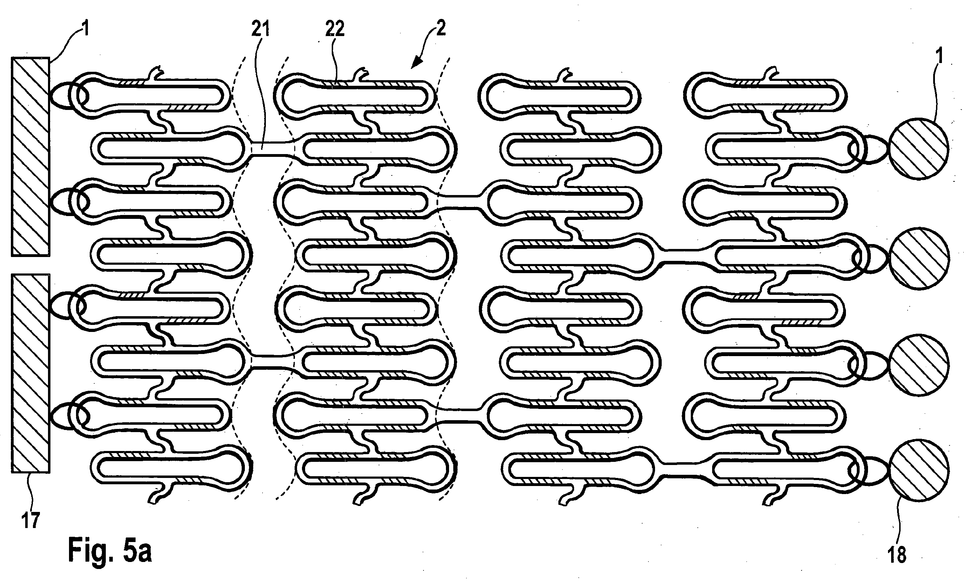

Erfindungsgemäße Ausgestaltungen für die vorstehend dargestellten bevorzugten Gegenstände werden beispielhaft in Figuren 3a und 5a dargestellt.

Ein weiterer Vorteil der erfindungsgemäßen Gegenstände besteht darin, dass mehrere Wirkstoffdepots, welche jeweils unterschiedliche Wirkstoffe umfassen, mit einem endovaskulär implantierbaren Körper verbunden werden können, und so ein multipte-drug-release (Freisetzung mehrerer Wirkstoffe), insbesondere dual-drug-release (Freisetzung von zwei Wirkstoffen) oder ein tripel-drug-realease (Freisetzung von drei Wirkstoffen) erreicht wird, was durch eine herkömmliche Beschichtungsverfahren technologisch kaum oder nur mit erhöhtem Kosten- und/oder Zeitaufwand erreicht werden kann. Ein Beispiel für eine solche erfindungsgemäße Ausgestaltung wird in Figur 2a beschrieben.

Ein weiterer Vorteil besteht darin, dass Interaktionen zwischen den verwendeten Wirkstoffen und dem verwendeten Material für den implantierbaren Körper, bevorzugt Stent, verringert werden kann. Dies wird insbesondere dadurch bewerkstelligt, dass der endovaskulär implantierbare Körper, bevorzugt Stent, getrennt von dem erfindungsgemäßen Wirkstoffdepot hergestellt wird. Dies ist insbesondere von Nutzen bei Verwendung von degradierbaren (Grund-)körpern, wie z.B. degradierbaren Stents, insbesondere degradierbaren Metallstents, da hier eine direkte Wirkstoffbeschichtung mit dem (Grund-)körper, aufgrund der Wechselwirkung mit dem Material des Körpers, mittels üblicher Verfahren nur schwierig bzw. teilweise überhaupt nicht möglich ist. Weiter bevorzugt ist die endständige Verknüpfung eines oder mehrerer Wirkstoffdepots an degradierbare Grundkörper, da der Abstand zwischen den sich wechselseitig beeinflussenden Materialien erhöht wird und damit die gegenseitige Beeinflussung verringert werden kann.

Die bevorzugten Ausgestaltungen der erfindungsgemäßen Gegenstände werden im Anschluss hieran sowie in den abhängigen Patentansprüchen beschrieben und sind des Weiteren der Figurenbeschreibung und den dazugehörigen Figuren zu entnehmen. Die bevorzugten Ausgestaltungen treffen auf alle Gegenstände der vorliegenden Erfindung auch insofern zu, als dass kein ausdrücklicher Verweis hierzu offenbart wird.

Ein implantierbarer Körper im Sinne der vorliegenden Erfindung stellt zum einen üblicherweise einen degradierbaren oder nicht degradierbaren, kardiovaskulären oder peripheren Stent sowie einen Stent für andere Hohlräume, wie beispielsweise die Speiseröhre, den Gallengang, die Harnröhre, die Prostata oder die Luftröhre dar. Zum anderen stellen implantierbare Körper im Sinne der vorliegenden Erfindung local drug delivery Implantate dar, die endovaskulär im Blut oder anderen Hohlräumen implantiert werden. Im Weiteren können implantierbare Körper Neuroapplikationen darstellen, sowie subkutane Anwendungen zum kontinuierlichen Wirkstoffrelease (beispielsweise Hormonpräparate), aber auch Stimulationselektronen oder regional drug delivery Applikationen.

Im Sinne der vorliegenden Erfindung umfassen degradierbare Körper, bevorzugt Stents, als Werkstoffe degradierbares Metall oder degradierbares Polymer.

### Degradierbarer Metallwerkstoff:

Vorzugsweise ist der degradierbare metallische Werkstoff eine biokorrodierbare Legierung, ausgewählt aus der Gruppe Magnesium, Eisen, Zink und Wolfram; insbesondere ist der degradierbare metallische Werkstoff eine Magnesiumlegierung.

Die Legierung, insbesondere umfassend Magnesium, Eisen, Zink und Wolfram, ist so in ihrer Zusammensetzung zu wählen, dass sie biokorrodierbar ist. Als biokorrodierbar im Sinne der vorliegenden Erfindung werden Legierungen bezeichnet, bei denen in physiologischer Umgebung ein Abbau stattfindet, der letztendlich dazu führt, dass der gesamte Stent oder der aus dem Werkstoff gebildete Teil des Stents seine mechanische Integrität verliert. Unter Legierung wird vorwiegend ein mechanisches Gefüge verstanden, deren Hauptkomponente Magnesium, Eisen, Zink oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, weiter bevorzugt mehr als 70 Gew.%.

Ist der Werkstoff eine Magnesiumlegierung, so enthält diese vorzugsweise Yttrium und weitere Seltenerdmetalle, da sich eine derartige Legierung aufgrund ihrer physikochemischen Eigenschaften und hohen Biokompatibilitäten insbesondere auch seiner Abbauprodukte, auszeichnet.

Besonders bevorzugt sind Legierungen der WE Reihe (WE 43 etc.) sowie Magnesiumlegierungen der Zusammensetzung Seltenerdmetalle 5,5 bis 9,9 Gew.%, davon Yttrium 0,0 bis 5,5 Gew.% und Rest umfassend Zirkon < 1 Gew.%, wobei Magnesium den auf 100 Gew.% fehlenden Anteil an den Legierungen einnimmt. Diese Magnesiumlegierungen bestätigten bereits experimentell und in ersten klinischen Versuchen ihre besondere Eignungen, d.h. sie zeigen eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorwiegend Scandium (21), Yttrium (37), Lanthan (57) und die 14 nach Lanthan (57) folgenden Elemente, nämlich Cer (58), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71), verstanden.

Insbesondere bei der Verbindung von erfindungsgemäßen Wirkstoffdepots mit Magnesiumstents können aufgrund des räumlichen Abstandes unerwünschte Wechselwirkungen des sauer degradierbaren polymeren Wirkstoffdepots und des basisch degradierenden Magnesium-Stents, insbesondere die daraus resultierende Beschleunigung der Magnesiumkorrosion und damit ein schnellerer Verlust des Stent-Kollapsdruckes, vermindert werden. Durch den vergrößerten räumlichen Abstand kann ebenfalls die Degradierung des Polymers in dem Wirkstoffdepot verlangsamt werden, wodurch weniger Reizungen des Gewebes auftreten und kein Abschälen der Polymerschicht von dem implantierten Stent auftritt.

### Degradierbare Polymerkörper:

Körper, insbesondere Stents, aus degradierbarem Polymer bestehen vorzugsweise aus Polydioxanon; Polyglycolid; Polycaprolacton; Polylactide, vorzugsweise Poly-L-lactid, Poly-D-L-lactid, Poly-D,L-lactid und Polymere sowie Blends, wie Poly-(L-lactid-CO-glycolid), Poly-(D,L-lactid-co-glycolid), Poly-(L-lactid-co-D,L-lactid), Poly-(L-lactid-co-trimethylencarbonat) und Tri-Blockcopolymere; Polysaccharide, vorzugsweise Chitosan, Levan, Hyaluronsäure, Heparin, Dextran und Cellulose; Polyhydroxyvalerat; Ethylvinylacetat; Polyethylenoxid; Polyphosphorylcholin; Fibrin; Albumin etc.

Werden im Sinne der vorliegenden Erfindung endovaskulär implantierbare Stents als implantierbare Körper verwendet, so können alle üblichen Stentgeometrien verwendet werden. Insbesondere bevorzugt sind Stentgeometrien, die in US 6,896,695, US 2006/241742, US 5,968,083 (Tenax), EP 1 430 854 (Helix-Design), US 6,197,047 und EP 0 884 985 beschrieben werden.

Vorzugsweise werden, sofern Stents als endovaskulär implantierbare Körper verwendet werden, das oder die Wirkstoffdepots nach Crimpung des Stents auf einen zu verwendenden Katheter mit diesem mechanisch verbunden. Vorteilhaft an dieser Reihenfolge ist, dass das oder die Wirkstoffdepots durch den Vorgang der Crimpung nicht in ihrer Matrix beeinträchtigt werden.

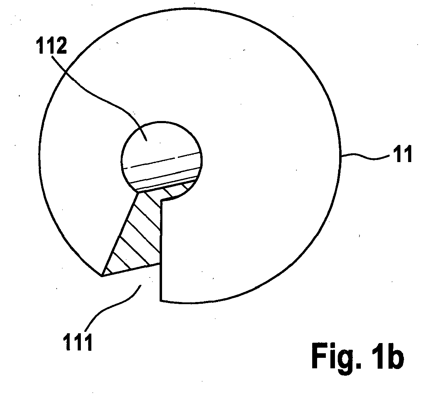

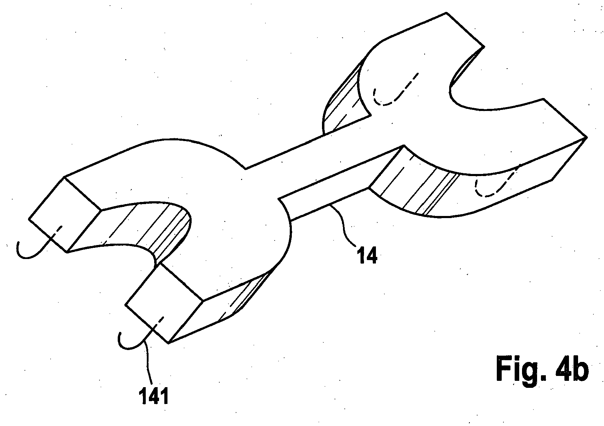

Die mechanische Verbindung des erfindungsgemäß hergestellten Wirkstoffdepots mit dem endovaskulär implantierbaren Körper, bevorzugt einem endovaskulären Stent, kann vorzugsweise formschlüssig mit einem Steg bzw. Strut eines Stents, ausgestaltet sein. Vorzugsweise mittels eines C-förmigen Greifers, der das Wirkstoffdepot mit dem implantierbaren Körper, bevorzugt einem Stent, vorzugsweise formschlüssig anklippt. Eine solche erfindungsgemäße Ausgestaltung wird beispielsweise in Figur 1b, 2c, 3b, 4c, 4e, 5b und 5c dargestellt. Aber auch alle anderen Befestigungsmaßnahmen der erfindungsgemäßen Stents, die der Fachmann aufgrund seiner Fachkenntnis in Betracht zieht, werden hiermit ebenfalls beansprucht. Hierunter fallen insbesondere Ausgestaltungen in Hakenform, welche beispielhaft in Figur 4b dargestellt werden.

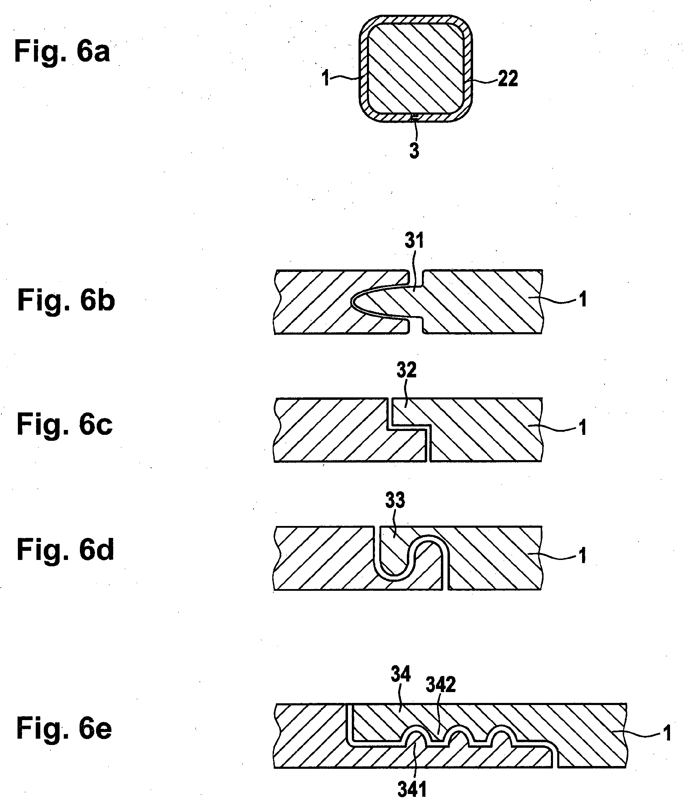

Denkbar sind ebenfalls Wirkstoffdepots, die eine Strebe bzw. einen Strut eines Stents vollständig umschließen und in geeigneter Weise miteinander so verbunden werden, dass das Wirkstoffdepot sich nicht von der Strebe bzw. dem Strut abrollen kann (siehe Figuren 6a bis 6e).

Pro implantierbarem Körper, vorzugsweise Stent, können ein oder mehrere Wirkstoffdepots, die sich gegebenenfalls in der Wirkstoffkonzentration und/oder in der Wirkstoffart unterscheiden, angeklippt werden. Die Wirkstoffdepots sind in der Regel so ausgestaltet, dass sie bei Implantation des Körpers in ein Gefäßsystem möglichst wenig stören und werden deshalb vorzugsweise in vorhandenen Zwischenräumen des implantierbaren Körpers, vorzugsweise des Stents, untergebracht oder sie überziehen den Körper, vorzugsweise den Stent, als dünne, wenig auftragende Schläuche. In einer weiteren bevorzugten Ausgestaltung weist lediglich die abluminale Oberfläche eines erfindungsgemäß wirkstoffbeladenen Körpers, bevorzugt Stents, das Wirkstoffdepot, insbesondere den Anteil des Wirkstoffdepots auf, der mit Wirkstoff beschichtet ist, auf.

Vorzugsweise werden das oder eines der Polymere des Wirkstoffdepots ausgewählt aus der Gruppe bestehend aus:
- nicht degradierbare Polymere: Polyethylen; Polyvinylchlorid; Polyacrylate; vorzugsweise Polyetyl- und Polymethylacrylate, Polymethylmetacrylat, Polymethyl-co-ethyl-acrylat und Ethylen/Ethylacrylat; Polytetrafluorethylen, vorzugsweise Ethylen/Chlortrifluorethylen Copolymere, Ethylen/Tretrafluorethylen Copolymere; Polyamide, vorzugsweise Polyamidimid, PA-11, PA-12, PA-46, PA-66; Polyetherimid; Polyethersulfon; Poly(iso)butylen; Polyvinylchlorid; Polyvinylfluorid; Polyvinylalkohol; Polyurethan; Polybuthylenterephthalat; Silikone; Polyphosphazen; Polymer-schäume, vorzugsweise Polymerschäume aus Carbonaten, Styrolen; Copolymere und/oder Blends der aufgezählten Polymerklassen, Polymere der Klasse der Thermoplaste sowie
- degradierbare Polymere: Polydioxanon; Polyglycolid; Polycaprolacton; Polylactide, vorzugsweise Poly-L-Lactid, Poly D,L Lactid, und Copolymere sowie Blends hiervon, vorzugsweise Poly(L-Lactid-co-glycolid), Poly(D,L-lactid-co-glycolid), Poly(L-Lactid-co-D,L-Lactid), Poly(I-Lactid-co-trimethylen carbonat); Triblockcopolymere; Polysaccharide, vorzugsweise Chitosan, Levan, Hyaluronsäure, Heparin, Dextran, Cellulose; Polyhydroxyvalerat; Ethylvinylacetat; Polyethylenoxid; Polyphosphorylcholin; Fibrin; Albumin; Polyhydroxybuttersäure, vorzugsweise ataktische, isotaktische und/oder syndiotaktische Polyhydroxybuttersäure sowie deren Blends.

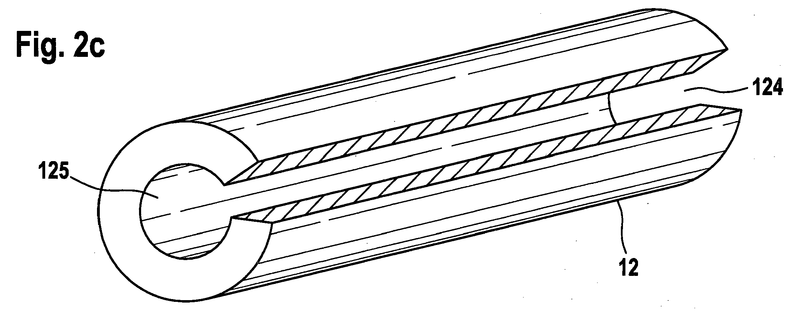

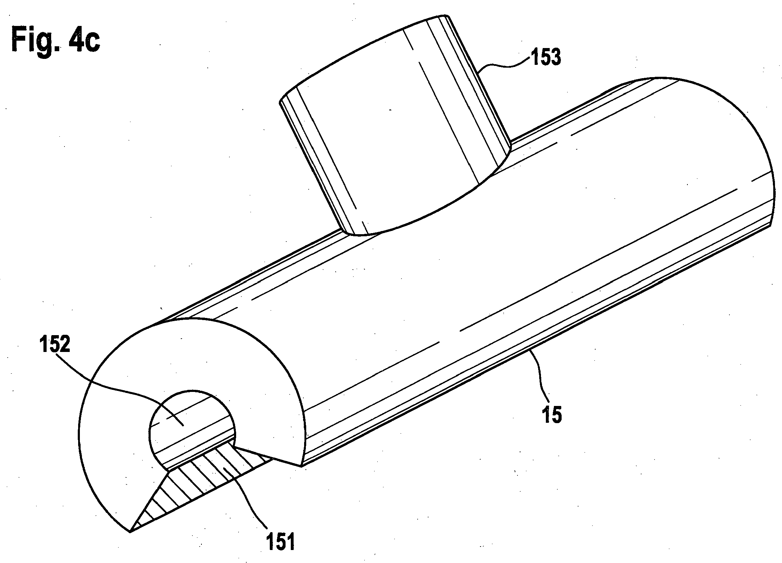

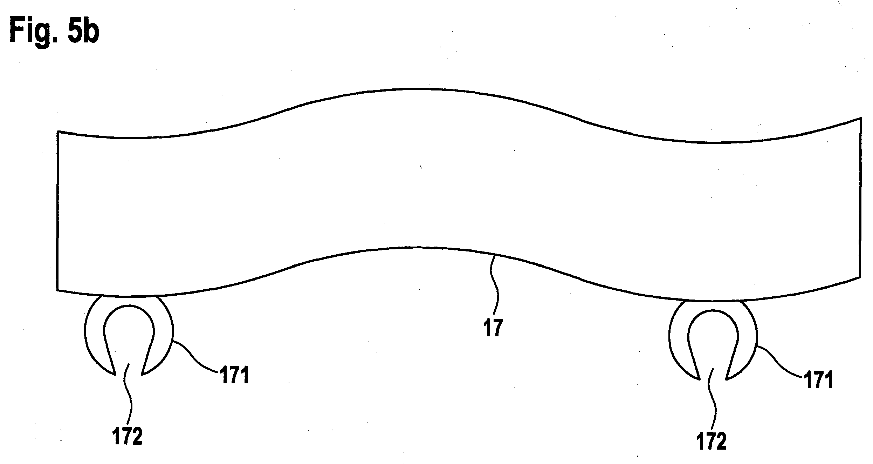

Vorzugsweise kennzeichnet sich ein erfindungsgemäß hergestelltes Wirkstoffdepot dadurch, dass es in Schlauchform (siehe beispielhaft Figuren 2c und 4c), Folienform (siehe beispielhaft Figur 5b), Preform (siehe beispielhaft Figuren 1b, 3b, 4b, 4e und 5c) oder als (relativ) steife Netze hergestellt wird.

Im Sinne der vorliegenden Erfindung bezeichnet der Begriff "Schlauch" einen im Querschnitt runden oder ovalen an zwei Seiten geöffneten, gegebenenfalls flexiblen Formkörper.

Im Sinne der vorliegenden Erfindung beschreibt der Begriff "Preform" eine Negativ-Form einer Stelle des Implantats, auf die sie später angebracht wird. Diese "Preform" kann gegebenenfalls flexibel oder aber auch steif sein.

Wird ein erfindungsgemäß hergestelltes Wirkstoffdepot in Schlauchform verwendet, so wird es vorzugsweise mittels eines reinen Extrusionsverfahrens, Schlauchblasverfahrens oder Tiefziehprozesses hergestellt. In seltenen Fällen aber auch über Füge- oder Klebetechnik. Unter Fügetechnik ist im Falle des Schlauches ein Aufschrumpfen oder ein "auf Passung" Zusammenfügen durch z.B. Aufstecken zu verstehen. Sofern der Schlauch nur einen Zwischenschritt bei der Produktion des Medikamententrägers darstellt, kann dieser auch geschlitzt, innenseitig mit einem Haftvermittler ausgekleidet und schließlich mit dem Stent, durch z.B. Abrollen, verbunden werden. Alternativ kann der Stent selber mit dem Haftvermittler ausgestattet werden und dann durch z.B. Abrollen mit dem geschlitzten Schlauch verbunden werden.

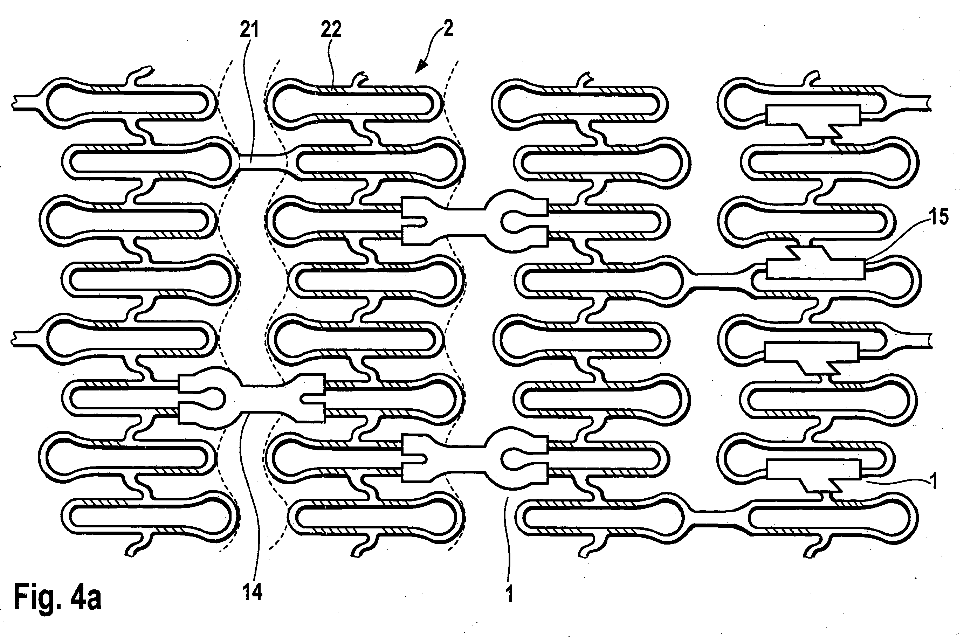

Wird ein erfindungsgemäß hergestelltes Wirkstoffdepot insbesondere zur mechanischen Verbindung mit einem endovaskulär implantierbaren Stent verwendet, so umfasst Verfahrensschritt c) des erfindungsgemäßen Verfahrens zusätzlich, dass das Wirkstoffdepot in Schlauchform auf die Länge eines Steges und/oder Struts des Stents gebracht wird und die Schlauchform längs der Achse aufgeschlitzt wird und der so bearbeitete Schlauch so ausgebildet ist, dass er einen Klipp darstellt, der mit den Stegen und/oder Struts des Stents mittels Krafteinwirkung vorzugsweise formschlüssig, mechanisch verbindbar ist. Eine solche Ausgestaltung wird insbesondere in Figuren 2a, 2b, 2c, 4a und 4c dargestellt.

Wird ein erfindungsgemäß hergestelltes Wirkstoffdepot in Folienform verwendet, so wird es vorzugsweise mittels eines Extrusionsverfahrens, Gießverfahrens oder Walzverfahrens hergestellt. Mitunter können Folien auch aus einer Schmelze oder Lösung gezogen werden.

Vorzugsweise umfasst das erfindungsgemäße Verfahren zur Herstellung des Wirkstoffdepots in Verfahrensschritt c) zusätzlich, dass das Wirkstoffdepot in Folienform so bearbeitet wird, dass die Folienform i) mit der Fläche der abluminalen Oberfläche des Körpers, bevorzugt des Stents oder ii) mit einem Teil davon verbunden wird. Eine Folie kann demnach um den gesamten Stent bzw. um eine Strebe bzw. Strut eines Stents angebracht werden und insbesondere wie in Figur 5a an die Streben an den Enden eines implantierbaren Stents angebracht werden. Sofern eine Folie an die Enden eines Implantates, bevorzugt eines Stents angebracht wird, sollte die Folie so beschaffen sein, dass sie nicht in das Lumen gerät und so den ungehinderten Blutstrom beeinträchtigt.

Wird das erfindungsgemäß hergestellte Wirkstoffdepot in Folienform zur Verbindung mit einem endovaskulär implantierbaren Stent verwendet, so kennzeichnet sich die Folienform i) oder ii) des erfindungsgemäß verwendeten Wirkstoffdepots bevorzugt dadurch, dass die Folienform entsprechend der Geometrie der abluminalen Oberfläche des Stents perforiert vorliegt kann, wobei das Folienmaterial den Bereichen des Materials des Stents entspricht. Die in Figuren 5a und 5b beschriebenen Folien könnten demnach auch perforiert vorliegen, beispielsweise um den Blutstrom in den Seitenästen von Blutgefäßen zu ermöglichen.

Erfindungsgemäße Wirkstoffdepots in Folienform können ebenfalls mittels geeigneter Mittel, bevorzugt C-förmiger Greifer, Haken etc., mit dem endovaskulär implantierbaren Körper, vorzugsweise formschlüssig, verbunden werden. Eine solche bevorzugte erfindungsgemäße Ausgestaltung ist insbesondere in Figuren 5a und 5b dargestellt.

Wird das erfindungsgemäße Wirkstoffdepot als Preform hergestellt, so kennzeichnet es sich bevorzugt dadurch, dass es einen Hohlkörper darstellt, der beispielsweise mittels eines Hohlkörperblasverfahrens oder Spritzgussverfahrens hergestellt wird, wobei der Preform das Negativ einer Stelle des Implantates, bevorzugt Stents, auf der sie angebracht werden soll, darstellt.

Wird gemäß einem erfindungsgemäßen Verfahren zur Herstellung des Wirkstoffdepots ein Preform hergestellt, so ist sie bevorzugt so ausgebildet, dass sie auf den Körper, bevorzugt Stent, zusammengeklickt (Fig. 6a bis 6e) werden kann und so mit dem Körper, bevorzugt Stent, vorzugsweise formschlüssig mechanisch verbindbar ist.

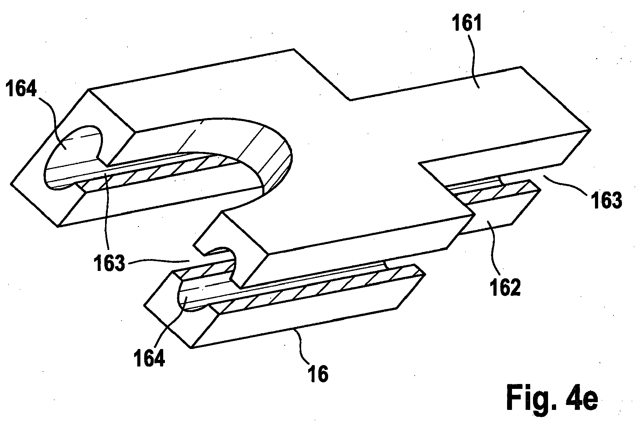

Wird ein Wirkstoffdepot als Preform für die Verbindung mit einem endovaskulär implantierbaren Stent verwendet, so wird es erfindungsgemäß bevorzugt so bearbeitet, dass das Preform längs der Achse aufgeschlitzt wird oder mit Schlitz bereits gegossen vorliegt und diese Preform so ausgebildet ist, dass sie einen Klipp darstellt, der mit dem Stent mittels Krafteinwirkung, vorzugsweise formschlüssig, mechanisch verbindbar ist (Fig. 4e).

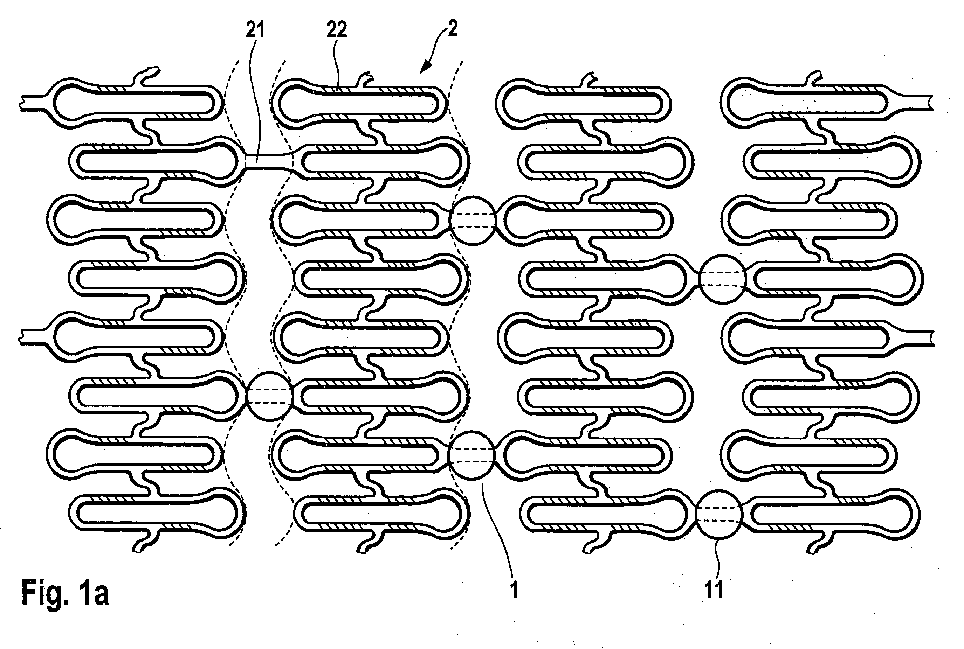

Wird ein Wirkstoffdepot als Preform zur Verbindung mit Struts bzw. Streben eines endovaskulär implantierbaren Stents verwendet, so wird das Preform gemäß des erfindungsgemäßen Verfahrens so bearbeitet, dass es auf die Länge der Stege bzw. Struts des Stents gebracht wird, längs der Achse aufgeschlitzt wird oder bereits mit Schlitz gegossen vorliegt und dieses Preform so ausgebildet ist, dass sie einen Klipp darstellt, der mit den Stegen bzw. Struts der Stents mittels Krafteinwirkung, vorzugsweise formschlüssig, mechanisch verbindbar ist. Eine solche erfindungsgemäße Ausgestaltung wird beispielhaft in Figuren 1a, 1b, 3b, 4c, 4e und 5c.

Alternativ kann das Preform aus zwei Teilen bestehen, die dann um den Strut bzw. die Strebe herum geschweißt werden.

Wirkstoffe, die bevorzugt für ein nach einem erfindungsgemäßen Verfahren hergestellten Wirkstoffdepot verwendet werden, eignen sich zur Prophylaxe oder Therapie einer In-Stent-Restenose oder Krebsbehandlung. Bevorzugt verwendete Wirkstoffe werden ausgewählt aus der Gruppe bestehend aus:
Lipidregulatoren, Immunsuppressiva, Vasodilatatoren, Calciumkanalblocker, Calcineurininhibitoren, Antiphlogistika, Antiinflammatorika, Antiallergika, Oligonucleotide, Estrogene, Endothelbildner, Steroide, Proteine, Peptide, Proliferationshemmer, Analgetika, Antirheumatika, Angiogeneseinhibitoren, Zytostatika.

Unter Zytostatika fallen insbesondere DNA alkylierende Substanzen, insbesondere Stickstoff-Lost-Verbindungen und Nitrosoharnstoffverbindungen; Platinverbindungen; Hydroxyharnstoffverbindungen; Antimetaboliten, vorzugsweise Folsäure-Antagonisten, Purinanaloga und Pyrimidinanaloga; Mikrotubuli-Inhibitoren, vorzugsweise Winker-Alkaloide, Taxane, bevorzugt Paclitaxel und Dozetaxel; Topoisomerase-Inhibitoren; Antibiotika, bevorzugt Anthracycline, insbesondere bevorzugt Danorobizin, Doxorubizin, Epirubizin und Idarubizin, Anactinomycine, insbesondere Dactinomycin, Methoxanthron, Asarkrin und Ansarkrin, Mitomycin C und Bleomycin; sowie verschiedenste Zytostatika aus der Gruppe Asparaginase, Metefusin und Imatinib; Hormone, bevorzugt Glucocorticoide, insbesondere Prednison, Sexualhormone, insbesondere bevorzugt Estrogene, Gestagene, Gonadoliberin (GnRH), Fludamid, Bizalutamid, Tamoxifen und Toremifen, Aromatasehemmstoffe, wie z.B. Aminoglutetimid, Formestan, Eksemistan, Retrozol und Anastrozol; Antikörper, Immunmodulatoren und Cytokine, bevorzugt Trastuzumab, Cetiximab, Rituximap, Alemtuzumab, Daklizumab, Gemtuzumab, Etratuzumab und Ibritumomap; Interleukin-II, Interferon-α, Tumor-Nekrose-Faktor-α (TNF-α), hämatopoetische Wachstumsfaktoren, wie z.B. G-CSF, GM-CSF.

Wird ein endovaskulär implantierbarer Körper, bevorzugt ein Stent, weiter bevorzugt ein degradierbarer Stent, mit einem nach einem erfindungsgemäßen Verfahren hergestellten Wirkstoffdepot, vorzugsweise formschlüssig, mechanisch verbunden, so umfasst das Verfahren vorzugsweise zusätzlich, dass der oder einer der Körper aus Schritt a) des erfindungsgemäßen Verfahrens vor Verbindung mit dem Wirkstoffdepot in Schritt c) mit einem oder mehreren Hilfsmitteln ganz oder teilweise beschichtet wird, wobei das oder die Hilfsmittel so ausgebildet sind, dass sie die mechanische Verbindung des oder der Wirkstoffdepots mit dem oder den Körpern verstärken.

Wird gemäß einer bevorzugten Ausgestaltung des erfindungsgemäßen Herstellungsverfahrens für die Herstellung des mit Wirkstoff beladenen endovaskulär implantierbaren Körper, bevorzugt eines (degradierbaren) Stents, eine Schlauchform als Wirkstoffdepot verwendet, so ist sie bevorzugt so ausgebildet, dass sie der Länge eines Stegs bzw. Struts des Stents entspricht, längs der Achse aufgeschlitzt ist und somit einen Klipp darstellt, der in Verfahrensschritt c) mittels Krafteinwirkung mit dem Steg bzw. Strut des Stents, vorzugsweise formschlüssig, mechanisch verbunden wird.

Wird ein erfindungsgemäß hergestelltes Wirkstoffdepot in Folienform verwendet, wobei bevorzugt Folie i) oder ii) verwendet wird, so wird der endovaskulär implantierbare Körper, bevorzugt ein (degradierbarer) Stent, in Schritt c) mit der abluminalen Oberfläche auf die Folie i) oder ii) abgerollt und somit mittels Krafteinwirkung, vorzugsweise formschlüssig, mechanisch verbunden wird.

Wird ein Preform verwendet, so kennzeichnet sich das erfindungsgemäße Herstellverfahren dadurch, dass das Preform so ausgebildet ist, dass es in Schritt c) auf den implantierbaren Körper, vorzugsweise Stent, zusammengeklickt wird und so mit dem Körper, vorzugsweise formschlüssig, mechanisch verbunden wird.

Alternativ kann das Preform auch durch Aufschrumpfen bei dezenter Wärmezufuhr an den implantierbaren Körper angebracht werden, damit der oder die Wirkstoffe nicht beschädigt werden.

Alternativ kennzeichnet sich ein erfindungsgemäßes Verfahren zur Herstellung des wirkstoffbeladenen endovaskulär implantierbaren Körpers dadurch, dass in einem weiteren Schritt das Preform so bearbeitet wird, dass sie längs der Achse aufgeschlitzt wird oder bereits mit Schlitz gegossen vorliegt und dieses Preform einen Klipp darstellt, der im Schritt c) mit dem Körper, vorzugsweise Stent, mittels Krafteinwirkung, vorzugsweise formschlüssig, mechanisch verbunden wird.

Vorzugsweise wird das Preform auf die Länge eines Steges oder eines Struts des Stents gebracht, längs der Achse aufgeschlitzt oder bereits mit Schlitz gegossen, so dass dieses Preform einen Klipp darstellt, der in Schritt c) mit dem Steg bzw. Strut des Stents mittels Krafteinwirkung, vorzugsweise formschlüssig, mechanisch verbunden wird.

Sofern der wirkstoffbeladene, endovaskulär implantierbare Körper einen, hergestellt nach einem erfindungsgemäßen Verfahren, Stent darstellt, so handelt es sich bevorzugt um einen degradierbaren endovaskulär implantierbaren Stent, besonders bevorzugt um einen degradierbaren Metallstent. Die zu dem erfindungsgemäßen Herstellverfahren hierin beschriebenen bevorzugten Ausgestaltungen sind auch auf den vorliegenden erfindungsgemäßen implantierbaren Körper, bevorzugt (degradierbarer) Stent, anzuwenden.

Sofern die vorliegende Erfindung einen Kit betrifft, werden die bevorzugten Ausgestaltungen hierzu, die sich auf das Wirkstoffdepot oder den endovaskulär implantierbaren Körper beziehen, durch die zu den erfindungsgemäßen Herstellverfahren beschriebenen bevorzugten Ausgestaltungen näher erläutert.

Bevorzugte Ausgestaltungen für die erfindungsgemäße Verwendung eines oder mehrerer Wirkstoffdepots zur Herstellung eines wirkstoffbeladenen implantierbaren Körpers oder für das erfindungsgemäße Verfahren zur Prophylaxe oder Behandlung einer Stenose, eines Aneurismas oder eines Tumorgewebe können dadurch erreicht werden, dass ein oder mehrere vorstehend genannte bevorzugte Ausgestaltungen in den Körper aufgenommen werden. Der oder die Wirkstoffe werden für die jeweilige Therapie speziell angebracht.

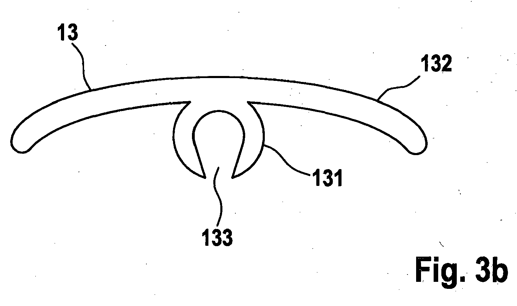

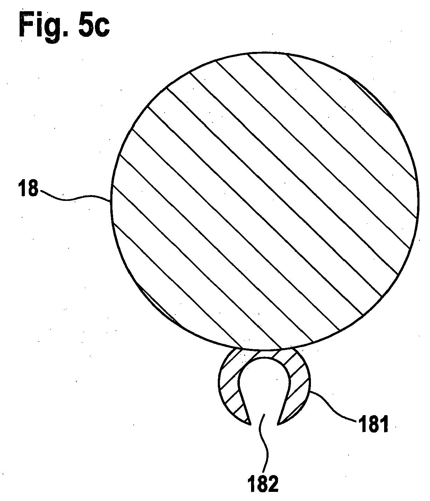

Für die Prophylaxe oder Therapie eines Tumorgewebes wird bevorzugt eine hohe Konzentration an Wirkstoff(en) verwendet. Demzufolge sind solche Wirkstoffdepotformen für eine diesbezüglich erfindungsgemäße Ausgestaltung bevorzugt, welche eine hohe Konzentration an Wirkstoff enthalten können. Beispielhaft werden geeignete Wirkstoffdepots in Figuren 3a, 3b, 5a, 5b und 5c gezeigt. Vorzugsweise wird ein erfindungsgemäß hergestellter, wirkstoffbeladener Stent in ein Blutgefäß, das Blut zum Tumor führt, bevorzugt nahe des Tumorgewebes, implantiert.

### Figurenbeschreibung:

Die Figuren zeigen beispielhaft Ausschnitte der abluminalen Oberfläche von Stentgeometrien eines Stentgrundkörpers bzw. die erfindungsgemäßen Wirkstoffdepots in perspektivischer Sicht oder im Querschnitt. Die vorliegende Erfindung wird allerdings nicht auf die hier gezeigten Stentgeometrien bzw. auf die gezeigte Anordnung der Wirkstoffdepots beschränkt.

Von den Figuren ist:
- Figur 1a:: Aufsicht auf einen abluminalen Ausschnitt einer Stentgeometrie 2 mit mehreren Wirkstoffdepots 1 in Perlenform 11.
- Figur 1 b:: Perspektivische Sicht auf ein geschlitztes Wirkstoffdepot in Perlenform 11.
- Figur 2a:: Aufsicht auf einen abluminalen Ausschnitt einer Stentgeometrie 2 mit mehreren Wirkstoffdepots 1 in Schlauchform 12.
- Figur 2b:: Aufsicht auf einen abluminalen Ausschnitt einer Stentgeometrie 2 mit mehreren Wirkstoffdepots 1 in Schlauchform 12 mit unterschiedlicher Beladung an Wirkstoffdepots über die Längsachse.
- Figur 2c:: Perspektivische Sicht auf ein geschlitztes Wirkstoffdepot in Schlauchform 12.
- Figur 3a:: Aufsicht auf einen abluminalen Ausschnitt einer Stentgeometrie 2 mit mehreren flächig geschlitzten Wirkstoffdepots 1, 13 angeklippt an Struts.
- Figur 3b:: Perspektivische Sicht auf ein flächig geschlitztes Wirkstoffdepot 13.
- Figur 4a:: Aufsicht auf einen abluminalen Ausschnitt einer Stentgeometrie 2 mit mehreren Wirkstoffdepots 1, 14 und 15, die auf der abluminale Oberfläche an Struts angeklippt werden.
- Figur 4b:: Perspektivische Sicht auf ein Wirkstoffdepot 14.
- Figur 4c:: Perspektivische Sicht auf ein Wirkstoffdepot 15.
- Figur 4d:: Aufsicht auf einen abluminalen Ausschnitt einer Stentgeometrie 2 mit mehreren Wirkstoffdepots 1 in geschlitzter Preform 16
- Figur 4e:: Perspektivische Sicht auf ein Wirkstoffdepot 16.
- Figur 5a:: Aufsicht auf einen abluminalen Ausschnitt einer Stentgeometrie 2 mit endständigen Wirkstoffdepots 1 in Form eines angeklippten Bandes 17 oder Körpers 18
- Figur 5b:: Perspektivische Sicht auf ein Wirkstoffdepot in Bandform 17
- Figur 5c:: Querschnitt eines Wirkstoffdepots in Form eines Körpers 18
- Figur 6a:: Querschnitt einer Stentstrebe 22 mit einem Wirkstoffdepot 1 mit Verbindungsmechanismus 3
- Figur 6b:: Schematische Darstellung des Verbindungsmechanismusses 31 des Wirkstoffdepots 1
- Figur 6c:: Schematische Darstellung des Verbindungsmechanismusses 32 des Wirkstoffdepots 1
- Figur 6d:: Schematische Darstellung des Verbindungsmechanismusses 33 des Wirkstoffdepots 1
- Figur 6e:: Schematische darstellung des Verbindungsmechanismusses 34 des Wirkstoffdepots 1

Figur 1 a zeigt einen abluminalen Ausschnitt einer Stentgeometrie 2, die in US 6,896,695 beschrieben wird, zusammen mit mehreren, vorzugsweise formschlüssig, angeklippten Wirkstoffdepots 1 in Form von geschlitzten Perlen 11, wobei die erfindungsgemäßen Wirkstoffdepotperlen 11 einen Hohlraum aufweisen und so geschlitzt 111 sind, dass die Perlen insbesondere an die Längsverbinder (Struts) 21 oder alternativ an die geraden Bereiche der Stentstreben 22 (letztere Ausführung nicht gezeigt), vorzugsweise formschlüssig, angeklippt werden können (siehe auch Figur 1 b). Eine solche erfindungsgemäße Ausgestaltung ist bevorzugt für ein oder mehrere Wirkstoffe, die nach Freisetzung des oder der Wirkstoffe eine gute Verteilung in dem Gefäßgewebe aufweisen.

Ein weiterer Vorteil besteht darin, dass durch den hohen Anteil eines nicht wirkstoffbeladenen Stentgrundkörpers 2 eine verbesserte Bewachsung mit Endothelialzellen (EC; Endothelialisierung) gegenüber üblicherweise vollständig beschichteter Stents aus dem Stand der Technik ermöglicht werden kann. Durch diese verbesserte Endothelialisierung des Stents 2 kann zudem das Risiko einer Thrombosenbildung, die mit der Implantation eines endovaskulär implantierbaren Körpers verbunden ist, vermindert werden.

Figur 1b zeigt eine Ansicht von erfindungsgemäßen Wirkstoffdepotperlen 11 mit einem Schlitz 111 und einem Hohlraum 112, der so ausgestaltet ist, dass er an einen Strut 21 oder Steg 22 eines Stents 2, vorzugsweise formschlüssig, angeklippt werden kann. Diese Wirkstoffdepotperlen 11 können erfindungsgemäß ein oder mehrere Wirkstoffe umfassen. Auch können die verwendeten Wirkstoffdepotperlen 11 jeweils unterschiedliche Konzentrationen eines oder mehrerer Wirkstoffe umfassen.

Erfindungsgemäße Wirkstoffdepots in Form von Perlen 11 werden bevorzugt hergestellt mittels Gieß-, Folien- und Fügeverfahren.

Figur 2a zeigt einen abluminalen Ausschnitt einer Stentgeometrie 2, die in US 6,896,695 beschrieben wird, mit mehreren angeklippten Wirkstoffdepots 1 in Schlauchform 12, wobei die Schläuche 12 so ausgestaltet sind, dass sie an der Längsseite entlang geschlitzt 126 sind und einen Hohlraum 127 aufweisen, um insbesondere an die Längsverbinder (Struts) 21 oder alternativ an die geraden Bereiche der Stentstreben 22 eines Stents 2, vorzugsweise formschlüssig, angeklippt werden zu können (siehe auch Figur 2c). Eine solche erfindungsgemäße Wirkstoffbeladung ist bevorzugt für eine flächendeckende Beladung mit zwei, drei oder mehreren Wirkstoffdepots (121, 122 und 123) interessant. Hierdurch kann ein multiple-drug release, insbesondere ein dual-drug-release (Freisetzung von 2 Wirkstoffen) oder ein triple-drug-release (Freisetzung von 3 Wirkstoffen) ermöglicht werden.

Eine solche Ausgestaltung ist insbesondere weiterhin bevorzugt für solche Wirkstoffdepots, deren Polymer mangels geeigneter Lösungsmittel oder Polymerhaftung auf dem verwendeten Stentmaterial nicht gemäß üblicher Beschichtungsverfahren mit dem Grundkörper des Stents oder des implantierbaren Körpers verbunden werden kann.

Figur 2b zeigt eine Abwandlung zu Figur 2a. Bei dieser erfindungsgemäßen Ausgestaltung weisen die Bereiche 23 des Stents eine niedrigere Beladung mit den Wirkstoffdepots 12, d.h. weniger Wirkstoffdepots 12 pro Oberfläche des Stents 2, gegenüber den Bereichen 24 des Stents auf. Die Wirkstoffdepots 12 sind vorzugsweise formschlüssig an den Steg 22 oder Strut 21 eines Stents 2 angeklippt.

Figur 2c zeigt eine Ansicht der geschlitzten erfindungsgemäßen Wirkstoffdepots in Schlauchform 12, einen Schlitz 124 und einen Hohlraum 125, der so ausgestaltet ist, dass er an einen Strut 21 oder Steg 22 eines Stents 2, vorzugsweise formschlüssig, angeklippt werden kann. Ein Wirkstoffdepot 12 kann üblicherweise ein oder mehrere Wirkstoffe enthalten. Wirkstoffdepots 121, 122 und 123 unterscheiden sich dadurch, dass sie jeweils unterschiedliche Wirkstoff umfassen.

Erfindungsgemäße Wirkstoffdepots in Schlauchform 12, 121, 122 und 123 werden bevorzugt mittels Extrusionsverfahren, Schlauchblasverfahren und Tiefziehprozess hergestellt. In seltenen Fällen werden Füge- oder Klebetechnik verwendet.

Figur 3a zeigt eine Aufsicht auf einen abluminalen Ausschnitt einer Stentgeometrie 2 mit mehreren flächig geschlitzten Wirkstoffdepots 13 in Preform, die an Struts 21 oder Stege 22 eines Stents 2, vorzugsweise formschlüssig, angeklippt sind.

In Figur 3b wird insbesondere dargestellt, dass das erfindungsgemäße Wirkstoffdepot 13 so ausgestaltet ist, dass es einen C-förmigen Greifer 131 mit Schlitz 133, der an einen Strut 21 oder Steg 22 eines Stents 2, vorzugsweise formschlüssig, angeklippt werden kann sowie eine Matrix 132 umfasst. Erfindungsgemäße Wirkstoffdepots 13 umfassen ein oder mehrere Wirkstoffe und werden bevorzugt mittels Gießverfahren, gegebenenfalls kombiniert mit Fräsen oder Zusammenstecken oder mittels eines geeigneten Fügeverfahrens (Heißkleben, Kleben) oder Spritzgußverfahrens, hergestellt.

Mittels Wirkstoffdepot 13 gemäß Figur 3a oder 3b wird eine hohe Wirkstoffkonzentration in dem Wirkstoffdepot 13 ermöglicht. Die Wirkstoffkonzentration wird bevorzugt in dem Matrixmaterial 132 des Wirkstoffdepots 13 lokalisiert, das auf der abluminalen Oberfläche des implantierbaren Körpers, bevorzugt Stents, angeordnet ist. Hierdurch wird ermöglicht, dass wenig bis kein Wirkstoff aus dem Wirkstoffdepot 13 in das Gefäßlumen von einem implantierten Stent 2 abgegeben wird und somit eine Endothelialisierung des Stents 2 nicht verzögert oder verhindert wird. Demzufolge wird erfindungsgemäß das Risiko einer Restenose oder einer Thrombosebildung verringert.

Figur 4a zeigt eine Aufsicht auf einen abluminalen Ausschnitt einer Stentgeometrie 2 mit mehreren Wirkstoffdepots 14 und 15, die auf der abluminale Oberfläche an Struts angeklippt werden. Diese erfindungsgemäße Ausgestaltung ist vergleichbar mit der erfindungsgemäßen Ausgestaltung in Figur 3a, mit dem Unterschied, dass die Wirkstoffdepots 14 und 15 im Gegensatz zu dem Wirkstoffdepot 13 nicht frei drehbar um die Struts 21 und Stege 22 des Stents 2 angeklippt sind. Durch eine erfindungsgemäße Ausgestaltung gemäß Figur 4a wird ermöglicht, dass mittels der erfindungsgemäßen Wirkstoffdepots 14 und 15 auf der luminalen Oberfläche der erfindugnsgemäß beladenen Stents 2 wenig Polymer und/oder Wirkstoff des jeweiligen Wirkstoffdepots 14 oder 15 vorhanden ist und somit eine Endothelialisierung des implantierten Stents 2 nicht vermindert oder verhindert wird. Dementsprechend sinkt das Risiko einer Restenose oder Thrombosebildung.

Figur 4b zeigt eine perspektivische Sicht auf ein erfindungsgemäßes Wirkstoffdepot 14. Dieses umfasst Mittel 141, die so ausgebildet sind, dass sie mit einem Strut 21 oder einem Steg 22 eines Stents 2, vorzugsweise formschlüssig, mechanisch verbindbar sind. Gemäß Figur 4b stellt das Mittel 141 insbesondere einen Hakenform dar. Diese erfindungsgemäßen Mittel können aus dem gleichen Material wie das Wirkstoffdepot hergestellt werden. Ein erfindungsgemäßes Wirkstoffdepot 14 ist weiterhin bevorzugt so ausgestaltet, dass es zumindest teilweise die abluminale Oberfläche eines Struts 21 und/oder Stegs 22 des Stents 2 in angeklippten Zustand bedeckt. Vorzugsweise ist an der luminalen Oberfläche des Stents 2 wenig bis kein Polymer, vorzugsweise kein wirkstoffhaltiges Polymer des Wirkstoffdepots 14 oder 15 vorhanden.

Figur 4c zeigt eine perspektivische Sicht auf ein erfindungsgemäßes Wirkstoffdepot 15. Wirkstoffdepot 15 ist in Schlauch bzw. Preform ausgestaltet, wobei es einen Schlitz 151 und einen Hohlraum 152 aufweist, der so ausgestaltet ist, dass er an einen Strut 21 oder Steg 22, vorzugsweise formschlüssig, angeklippt werden kann. Ferner umfasst das Wirkstoffdepot 15 Material, dass so ausgestaltet ist, dass es in angeklipptem Zustand die abluminale Oberfläche eines Struts 21 oder Stegs 22 bedeckt. In einer bevorzugten Ausgestaltung ist der Schlitz 151 so breit, dass das Wirkstoffdepot 15 in an den Strut 21 oder Steg 22 des Stents 2 angeklipptem Zustand auf der luminalen Seite wenig oder kein Matrixmaterial des Wirkstoffdepots 15 aufweist.

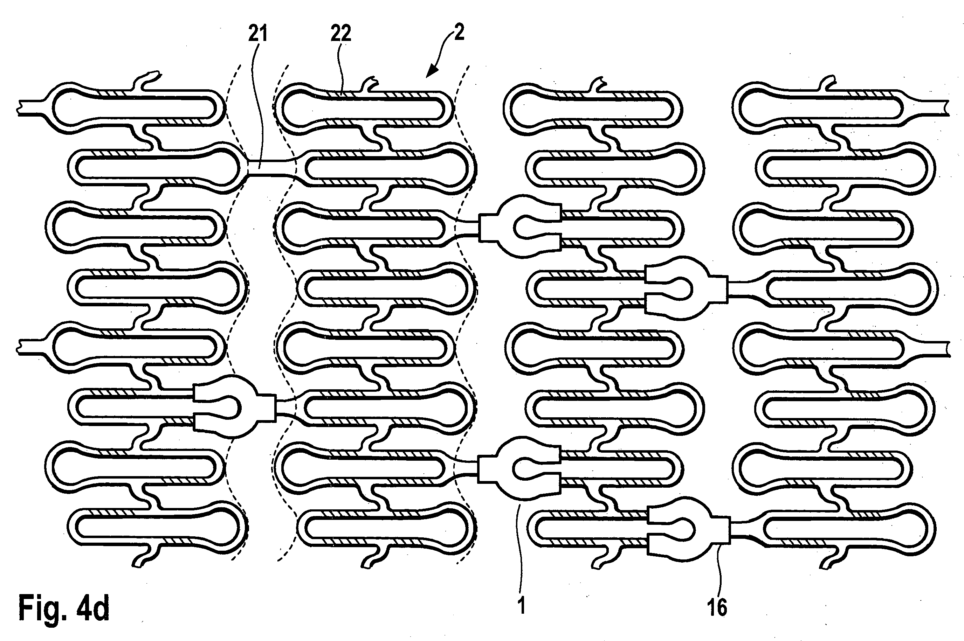

Figur 4d zeigt eine Aufsicht auf einen abluminalen Ausschnitt einer Stentgeometrie 2 mit mehreren Wirkstoffdepots 16. Die Wirkstoffdepots sind so ausgestaltet, dass sie in angeklipptem Zustand sowohl luminale als auch die entsprechenden abluminalen Bereiche eines oder mehrerer Stege 22 und/oder Struts 21 eines Stents 2, vorzugsweise formschlüssig, bedecken.

Figur 4e zeigt eine perspektivische Sicht auf ein Wirkstoffdepot 16, dass so ausgestaltet ist, dass es in angeklipptem Zustand sowohl luminale als auch die entsprechenden abluminalen Bereiche eines oder mehrerer Stege 22 und/oder Struts 21 eines Stents 2, vorzugsweise formschlüssig, bedeckt. Hierfür weist das Wirkstoffdepot 16 eine oberen Bereich 161 auf, der vorzugsweise die abluminale Oberfläche eines Stents 2 bedeckt, und einen unteren Bereich 162 auf, der vorzugsweise die luminale Oberfläche eines Stents 2 bedeckt. Zudem weist ein erfindungsgemäßes Wirkstoffdepot 16 einen Schlitz 163 auf, der ermöglicht, dass das Wirkstoffdepot 16 an einen oder mehrere Stege 22 und Struts 21 geklippt werden kann. Vorzugsweise umfasst ein erfindungsgemäßes Wirkstoffdepot Hohlräume 164, die so ausgebildet sind, dass die im angeklippten Zustand, vorzugsweise formschlüssig, die Stege 22 und Struts 21 eines Stents 2 umfassen. Vorzugsweise sind lediglich im Bereich 161 des Wirkstoffdepots ein oder mehrere Wirk-Stoffe inkorporiert. Dies hat den Vorteil, dass durch eine verminderte Abgabe das oder der Wirkstoffe an das Gefäßlumen, die Endotheliasierung des Stents 2 nicht vermindert oder verhindert wird.

Figur 5a zeigt eine Aufsicht auf einen abluminalen Ausschnitt einer Stentgeometrie 2 mit jeweils endständig, vorzugsweise formschlüssig, angeklippten Wirkstoffdepots in Form eines Bandes 17 oder eines Körpers 18. Eine solche erfindungsgemäße Ausgestaltung wird seltener für einen anti-proliferativen Einsatz eines drug-eluting-stents (DES) eingesetzt, sondern bevorzugt für die Freisetzung von Wirkstoffen, insbesondere ausgewählt aus der Gruppe der Zytostatika, in die Blutbahn zu Behandlung von Tumoren. In dieser erfindungsgemäßen Ausgestaltung wird der Stent nicht seiner Tragstruktur wegen verwendet, sondern dient als Verankerung für die erfindungsgemäßen Wirkstoffdepots 17 und 18. Ein in dieser Weise erfindungsgemäß beladener Stent wird vorzugsweise in ein Blutgefäß implantiert, das Blut zu dem Tumorgewebe führt, bevorzugt nah an das Tumorgewebe. Hierdurch wird ermöglicht, dass eine möglichst hohe Konzentration an verwendetem Wirkstoff das Tumorgewebe erreicht.

Figur 5b zeigt eine perspektivische Sicht auf ein erfindungsgemäßes Wirkstoffdepot in Bandform 17. Das Band 17 weist zudem Mittel 171 auf, die ermöglichen, dass das Band 17 an einen endovaskulär implantierbaren Körper, bevorzugt Stent, mechanisch verbunden werden kann. Bevorzugt wird ein Mittel 171 in Form einen C-förmigen Greifers verwendet, der einen Schlitz 171 aufweist. In angeklipptem Zustand umfasst der C-förmige Greifer 171, vorzugsweise formschlüssig, einen Steg 22 oder einen Strut 21 eines Stents 2. Dieses Wirkstoffdepot 17 kann ein oder mehrere Wirkstoffe umfassen.

Figur 5c zeigt einen Querschnitt eines Wirkstoffdepots in Form eines Körpers 18. Der Körper 18 weist zudem Mittel 181 auf, die ermöglichen, dass der Körper 18 an einen endovaskulär implantierbaren Körper, bevorzugt Stent, mechanisch verbunden werden kann. Bevorzugt wird ein Mittel 181 in Form einen C-förmigen Greifers verwendet, der einen Schlitz 181 aufweist. In angeklipptem Zustand umfasst der C-förmige Greifer 181, vorzugsweise formschlüssig, einen Stege 22 oder einen Strut 21 eines Stents 2. Dieses Wirkstoffdepot 18 kann ein oder mehrere Wirkstoffe umfassen. Die C-förmigen Greifer können beispielsweise mittels Spritzguss, Reservoir, durch Fügeverfahren (Kleben, Aufschmelzen) oder nach geeigneten anderen vorstehend beschriebenen Verfahren hergestellt werden.

Figur 6a zeigt einen Querschnitt einer Stentstrebe 22 mit einem die Stentstrebe umgebenes Wirkstoffdepot 1 und Verbindungsmechanismus 30.

In Figur 6b wird der Verbindungsmechanismus 31 des Wirkstoffdepots 1 vergrößert dargestellt. Bei dem Verbindungsmechanismus 31 handelt es sich bei dem einen Ende des Wirkstoffdepots um eine Einkerbung, die ggf. durch Fräsen in das Wirkstoffdepot eingebracht werden kann und bei dem gegenüberliegendem zweiten Ende des Wirkstoffdepots um eine Ausstülpung, die geeignet ist in die Einkerbung passend gesteckt zu werden, und so eine feste Verbindung der beiden Enden des Wirkstoffdepots zu bewirken.

In Figur 6c wird der Verbindungsmechanismus 32 des Wirkstoffdepots 1 vergrößert dargestellt. Bei dem Verbindungsmechanismus 32 handelt es sich um einen Verbindungsmechanismus nach L-Form, wobei die sich gegenüberliegenden Enden der Wirkstoffdepots jeweils spiegelverkehrte L-förmige Enden aufweisen, die passend aufeinander gesteckt werden können und so eine feste Verbindung der Enden des Wirkstoffdepots 1 bewirken.

In Figur 6d wird ein Verbindungsmechanismus 33 des Wirkstoffdepots 1 vergrößert dargestellt. Der Verbindungsmechanismus 33 kennzeichnet sich dadurch, dass die beiden gegenüberliegenden Enden des Wirkstoffdepots 1 jeweils in Hakenform vorliegen und ineinander eingehakt werden und so eine feste Verbindung der Enden des Wirkstoffdepots 1 bewirken.

In Figur 6e wird ein Verbindungsmechanismus 34 des Wirkstoffdepots 1 vergrößert dargestellt. Der Verbindungsmechanismus 34 entspricht im Prinzip dem Verbindungsmechanismus in L-Form, wie bereits in Figur 6c beschrieben, jedoch unterscheidet sich der Verbindungsmechanismus 34 dadurch, dass die aufeinanderliegenden Fläche 341 Ausstülpungen aufweist und die Fläche 342 entsprechende Einkerbungen aufweist und so eine feste Verbindung der Enden des Wirkstoffdepots 1 bewirken.

## Patentansprüche

1. Verfahren zur Herstellung eines Wirkstoffdepots, das zur mechanischen Verbindung mit einer Oberfläche eines endovaskulär implantierbaren Körpers ausgebildet ist, umfassend oder bestehend aus folgenden Schritten:
a) Bereitstellung eines oder mehrerer Polymere,
b) Bereitstellung eines oder mehrerer Wirkstoffe und
c) Herstellung eines Wirkstoffdepots aus dem oder den Polymeren und dem oder den Wirkstoffen, wobei das Wirkstoffdepot so ausgebildet ist, dass es mittels Krafteinwirkung mit der Oberfläche des Körpers mechanisch verbindbar ist, in der Weise, dass das Wirkstoffdepots an die Oberfläche des Körpers angeklippt wird.

2. Verfahren gemäß Anspruch 1, **dadurch gekennzeichnet, dass** das oder eines der Polymere nicht-degradierbare oder degradierbare Polymere darstellt und dass das oder eines der Polymere ausgewählt wird aus der Gruppe, bestehend aus:
- nicht degradierbare Polymere: Polyethylen; Polyvinylchlorid; Polyacrylate; vorzugsweise Polyetyl- und Polymethylacrylate, Polymethylmetacrylat, Polymethyl-co-ethyl-acrylat und Ethylen/Ethylacrylat; Polytetrafluorethylen, vorzugsweise Ethylen/Chlortrifluorethylen Copolymere, Ethylen/Tretrafluorethylen Copolymere; Polyamide, vorzugsweise Polyamidimid, PA-11, PA-12, PA-46, PA-66; Polyetherimid; Polyethersulfon; Poly(iso)butylen; Polyvinylchlorid; Polyvinylfluorid; Polyvinylalkohol; Polyurethan; Polybuthylenterephthalat; Silikone; Polyphosphazen; Polymerschäume, vorzugsweise Polymerschäume aus Carbonaten, Styrolen; Copolymere und/oder Blends der aufgezählten Polymerklassen, Polymere der Klasse der Thermoplaste sowie
- degradierbare Polymere: Polydioxanon; Polyglycolid; Polycaprolacton; Polylactide, vorzugsweise Poly-L-Lactid, Poly D,L Lactid, und Copolymere sowie Blends hiervon, vorzugsweise Poly(L-Lactid-co-glycolid), Poly(D,L-lactid-co-glycolid), Poly(L-Lactid-co-D,L-Lactid), Poly(1-Lactid-co-trimethylen carbonat); Triblockcopolymere; Polysaccharide, vorzugsweise Chitosan, Levan, Hyaluronsäure, Heparin, Dextran, Cellulose; Polyhydroxyvalerat; Ethylvinylacetat; Polyethylenoxid; Polyphosphorylcholin; Fibrin; Albumin; Polyhydroxybuttersäure, vorzugsweise ataktische, isotaktische und/oder syndiotaktische Polyhydroxybuttersäure sowie deren Blends.

3. Verfahren gemäß einem der vorstehenden Ansprüche, **dadurch gekennzeichnet, dass** das oder eines der Wirkstoffdepots in Schritt c) in Schlauchform, Folienform, Preform oder als Netz hergestellt wird, die Schlauchform mittels eines reinen Extrusionsverfahrens, Schlauchblasverfahren oder Tiefziehprozess hergestellt wird und das Wirkstoffdepot in Folienform mittels eines Extrusionsverfahrens, Gießverfahrens oder Walzverfahrens hergestellt wird.

4. Verfahren gemäß Anspruch 3, zusätzlich umfassend in Verfahrensschritt c), dass das Wirkstoffdepot in Schlauchform auf die Länge eines Steges bzw. Struts des Stents gebracht wird, die Schlauchform längs der Achse aufgeschlitzt wird und der so bearbeitete Schlauch so ausgebildet ist, dass er einen Klipp darstellt, der mit den Stegen oder bzw. den Struts des Stents mittels Krafteinwirkung mechanisch verbindbar ist.

5. Verfahren gemäß Anspruch 3, **dadurch gekennzeichnet, dass** das Wirkstoffdepot ein Preform einen Hohlkörper in Schlauchform darstellt, der mittels eines Hohlkörperblasverfahren oder Spritzgussverfahren hergestellt wird, wobei das Preform das Negativ einer Stelle des Stents oder Steges bzw. Struts des Stents darstellt, an die sie angebracht wird oder dass das Preform so ausgebildet ist, dass sie auf den Körper, bevorzugt Stent zusammengeklickt wird und so mit dem Körper, bevorzugt Stent mechanisch verbindbar ist.

6. Verfahren gemäß Anspruch 5, **dadurch gekennzeichnet, dass** das Preform bereits geschlitzt vorliegt oder in einem weiteren Schritt so bearbeitet wird, dass sie längs der Achse aufgeschlitzt wird, und dieses Preform so ausgebildet ist, dass es einen Klipp darstellt, der mit dem Stent mittels Krafteinwirkung mechanisch verbindbar ist oder dass das Preform auf die Länge der Stege bzw. Struts des Stents gebracht wird, geschlitzt vorliegt oder längs der Achse aufgeschlitzt wird und dieses Preform so ausgebildet ist, dass es einen Klipp darstellt, der mit den Stegen bzw- Struts der Stents mittels Krafteinwirkung mechanisch verbindbar ist.

7. Verfahren gemäß einem der vorstehenden Ansprüche, **dadurch gekennzeichnet, dass** der oder die Wirkstoffe geeignet sind zur Prophylaxe oder Therapie einer In-Stent-Restenose oder Krebsbehandlung und dass der oder die Wirkstoffe ausgewählt werden
aus der Gruppe bestehend aus:
Lipidregulatoren, Immunsuppressiva, Vasodilatatoren, Calciumkanalblocker, Calcineurininhibitoren, Antiphlogistika, Antiinflammatorika, Antiallergika, Oligonucleotide, Estrogene, Endothelbildner, Steroide, Proteine, Peptide, Proliferationshemmer, Analgetika, Antirheumatika, Angiogeneseinhibitoren, Zytostatika.

8. Verfahren zur Herstellung eines wirkstoffbeladenen endovaskulär implantierbaren Körpers, umfassend oder bestehend aus folgenden Schritten:
a) Bereitstellung eines endovaskulär implantierbaren Körpers,
b) Bereitstellung eines oder mehrerer unterschiedlicher Wirkstoffdepots, das oder die gemäß einer der Ansprüche 1 bis 7 hergestellt sind und
c) Mechanische Verbindung des oder eines der Wirkstoffdepots aus Schritt b) mit dem Körper aus Schritt a), in der Weise, dass das Wirkstoffdepots an die Oberfläche des Körpers angeklippt wird.

9. Verfahren gemäß Anspruch 8, zusätzlich umfassend, dass der oder einer der Körper aus Schritt a) vor Verbindung mit dem Wirkstoffdepot in Schritt c) mit einem oder mehreren Hilfsmitteln ganz oder teilweise beschichtet wird, wobei das oder die Hilfsmittel so ausgebildet sind, dass sie die mechanische Verbindung des oder der Wirkstoffdepots mit dem oder den Körper verstärken.

10. Verfahren gemäß Anspruch 8 oder 9, **dadurch gekennzeichnet, dass** das oder einer der Wirkstoffdepots in Schlauchform und so ausgebildet ist, dass sie der Länge eines Stegs bzw. Struts des Stents entspricht, längs der Achse aufgeschlitzt ist und somit einen Klipp darstellt, der in Verfahrensschritt c) mittels Krafteinwirkung mit dem Steg bzw. Strut des Stents mechanisch verbunden wird.

11. Verfahren gemäß Anspruch 8 oder 9, **dadurch gekennzeichnet, dass** das oder die Wirkstoffdepots in Folienform ausgebildet und i) mit der Fläche der abluminalen Oberfläche des Stents oder ii) einem Teil davon verbunden sind oder dass die Folienform i) oder ii) zusätzlich entsprechend der Geometrie der abluminalen Oberfläche des Stents perforiert vorliegt, wobei das Folienmaterial den Bereichen des Materials des Stents entspricht oder dass der Körper, bevorzugt Stent in Schritt c) mit der abluminalen Oberfläche auf die Folie i) oder ii) abgerollt wird und mit mittels Krafteinwirkung mechanisch verbunden wird.

12. Verfahren gemäß Anspruch 8 oder 9, **dadurch gekennzeichnet, dass** das oder einer der Wirkstoffdepots ein Preform einen Hohlkörper in Schlauchform darstellt, der mittels eines Hohlkörperblasverfahren oder Spritzgussverfahren hergestellt wird, wobei das Preform, eine negative Stelle des Stents oder des Steges bzw. Struts darstellt oder dass das Preform so ausgebildet ist, dass es in Schritt c) auf den Stent zusammengeklickt wird und so mit dem Stent mechanisch verbunden wird.

13. Verfahren gemäß Anspruch 12, **dadurch gekennzeichnet, dass** das Preform geschlitzt vorliegt oder in einem weiteren Schritt so bearbeitet wird, dass es längs der Achse aufgeschlitzt wird, und dieses Preform einen Klipp darstellt, der in Schritt c) mit dem Stent mittels Krafteinwirkung mechanisch verbunden wird oder dass in einem weiteren Schritt das Preform so bearbeitet wird, dass es auf die Länge eines Steges bzw. Struts des Stents gebracht wird, geschlitzt vorliegt oder längs der Achse aufgeschlitzt wird und diese Preform einen Klipp darstellt, der in Schritt c) mit dem Stegen oder Strut des Stents mittels Krafteinwirkung mechanisch verbunden wird.

14. Wirkstoffbeladener, endovaskulär implantierbarer Körper hergestellt nach einem der Verfahren gemäß einem der Ansprüche 8 bis 13.

15. Kit umfassend oder bestehend aus:
a) einem oder mehreren Wirkstoffdepots hergestellt nach einem der Ansprüche1 bis 7 und
b) einem oder mehreren endovaskulär implantierbaren Körpern.

16. Kit gemäß Anspruch 15, **dadurch gekennzeichnet, dass** der oder einer der Körper mit einem oder mehreren Hilfsmitteln ganz oder teilweise beschichtet ist, wobei das oder die Hilfsmittel so ausgebildet sind, dass sie die mechanische Verbindung des oder der Wirkstoffdepots mit dem oder den implantierbaren Körper verstärken.

17. Verwendung eines oder mehrerer Wirkstoffdepots hergestellt nach einem der Ansprüche 1 bis 7 zur Herstellung eines wirkstoffbeladenen, endovaskulär implantierbaren Körpers zu Prophylaxe oder Behandlung einer Stenose, eines Aneurisma oder eines Tumorgewebes in einem menschlichen oder tierischen Körper.

## Claims

1. A method for producing an active ingredient depot for mechanical connection to a surface of an endovascularly implantable body, comprising or consisting of the following steps:
a) providing one or more polymers;
b) providing one or more active ingredients; and
c) producing an active ingredient depot from said one or more polymers and said one or more active ingredients, wherein the active ingredient depot is formed such that it is mechanically connectable to the surface of the body by means of force application, such that the active ingredient depot is clipped onto the surface of the body.

2. The method according to Claim 1, **characterised in that** the polymer or one of the polymers represents non-degradable or degradable polymers, and **in that** the polymer or one of the polymers is selected from the group consisting of:
- non-degradable polymers: polyethylene; polyvinylchloride; polyacrylates; preferably polyethyl- and polymethylacrylates, polymethylmethacrylate, polymethyl-co-ethyl-acrylate, and ethylene/ethyl acrylate; polytetrafluoroethylene, preferably ethylene/chlorotrifluoroethylene copolymers, ethylene/tetrafluoroethylene copolymers; polyamides, preferably polyamide imide, PA-11, PA-12, PA-46, PA-66; polyetherimide; polyethersulfone; poly(iso)butylene; polyvinylchloride; polyvinylfluoride; polyvinylalcohol; polyurethane; polybutylene terephthalate; silicones; polyphosphazene; polymer foams, preferably polymer foams made of carbonates, styrenes; copolymers and/or blends of the above-listed polymer classes, polymers of the class of thermoplastics, and
- degradable polymers: polydioxanone; polyglycolide; polycaprolactone; polylactides, preferably poly-L-lactide, poly-D,L-lactide, and copolymers and blends thereof, preferably poly(L-lactide-co-glycolide), poly(D,L-lactide-co-glycolide), poly(L-lactide-co-D,L-lactide), poly(L-lactide-co-trimethylene carbonate); triblock copolymers; polysaccharides, preferably chitosan, levan, hyaluronic acid, heparin, dextran, cellulose; polyhydroxyvalerate; ethylvinylacetate; polyethylene oxide; polyphosphorylcholine; fibrin; albumin; polyhydroxy butyric acid, preferably atactic, isotactic, and/or syndiotactic polyhydroxy butyric acid and their blends.

3. The method according to one of the preceding claims, **characterised in that** the active ingredient depot or one of the active ingredient depots is produced in step c) in tube form, film form, preform, or as a net, the tube form is produced by way of a pure extrusion method, blow moulding method, or deep-drawing process, and the active ingredient depot in film form is produced by way of an extrusion method, casting method, or rolling method.

4. The method according to Claim 3, additionally comprising, in method step c), that the active ingredient depot in tube form is brought to the length of a bar or strut of the stent, the tube form is slit along the axis, and the tube thus processed is formed such that it represents a clip that is mechanically connectable to the bars or the struts of the stent by means of force application.

5. The method according to Claim 3, **characterised in that** the active ingredient depot is a preform a hollow body in tube form, produced by means of a hollow body blowing method or an injection-moulding method, wherein the preform represents the negative of a point of the stent or bar or strut of the stent to which it is attached, or **in that** the preform is formed such that it is clicked together onto the body, preferably a stent, and is thus mechanically connectable to the body, preferably a stent.

6. The method according to Claim 5, **characterised in that** the preform is already slit or is processed in a further step such that it is slit along the axis, and said preform is formed such that it represents a clip that is mechanically connectable to the stent by means of force application, or **in that** said preform is brought to the length of the bar or the strut of the stent, is already slit, or is slit along an axis, and said preform is formed such that it represents a clip that is mechanically connectable to the bars or struts of the stent by means of force application.

7. The method according to one of the preceding claims, **characterised in that** the active ingredient(s) is/are suitable for prophylaxis or therapy of an in-stent restenosis or cancer treatment, and **in that** the active ingredient(s) is/are selected from the group consisting of:
lipid regulators, immunosuppressives, vasodilators, calcium channel blockers, calcineurin inhibitors, antiphlogistics, anti-inflammatory agents, antiallergy agents, oligonucleotides, oestrogens, endothelium producers, steroids, proteins, peptides, proliferation inhibitors, analgesics, antirheumatics, angiogenesis inhibitors, and cytostatics.

8. A method for producing an active-ingredient-charged endovascularly implantable body, comprising or consisting of the following steps:
a) providing an endovascularly implantable body;
b) providing one or more different active ingredient depots, said active ingredient depot(s) being produced in accordance with one of Claims 1 to 7; and
c) mechanical connection of the active ingredient depot or one of the active ingredient depots from step b) to the body from step a), in such a way that the active ingredient depot is clipped onto the surface of the body.

9. The method according to Claim 8, additionally comprising that the body or one of the bodies from step a) is entirely or partially coated with one or more auxiliary agents before connection to the active ingredient depot in step c), wherein the auxiliary agent(s) is/are formed such that it/they reinforces/reinforce the mechanical connection of the active ingredient depot(s) to the body/bodies.

10. The method according to Claim 8 or 9, **characterised in that** the active ingredient depot or one of the active ingredient depots is provided in tube form and is formed such that it corresponds to the length of a bar or strut of the stent, is slit along the axis, and thus represents a clip that is mechanically connected to the bar or strut of the stent in method step c) by means of force application.

11. The method according to Claim 8 or 9, **characterised in that** the active ingredient depot(s) is/are formed in film form and i) is/are connected to the area of the abluminal surface of the stent or ii) part thereof, or **in that** the film form i) or ii) is additionally present in perforated form in accordance with the geometry of the abluminal surface of the stent, wherein the film material corresponds to the areas of the material of the stent, or **in that** the body, preferably the stent, is unrolled with the abluminal surface on the film i) or ii) in step c) and is mechanically connected by means of force application.

12. The method according to Claim 8 or 9, **characterised in that** the active ingredient depot or one of the active ingredient depots represents a preform a hollow body in tube form that is produced by means of a hollow body blowing method or an injection-moulding method, wherein the preform represents a negative point of the stent or of the bar or strut, or **in that** the preform is formed such that it is clicked together in step c) onto the stent and is thus mechanically connected to the stent.

13. The method according to Claim 12, **characterised in that** the preform is already slit or is processed in a further step such that it is slit along the axis, and this preform represents a clip that is mechanically connected in step c) to the stent by means of force application, or **in that** in a further step the preform is processed such that it is brought to the length of a bar or strut of the stent and is already slit or is slit along the axis, and said preform represents a clip that is mechanically connected in step c) to the bar or strut of the stent by means of force application.

14. An active-ingredient-charged, endovascularly implantable body produced in accordance with one of the methods according to one of Claims 8 to 13.

15. A kit, comprising or consisting of:
a) one or more active ingredient depots produced in accordance with one of Claims 1 to 7, and
b) one or more endovascularly implantable bodies.

16. The kit according to Claim 15, **characterised in that** the body or one of the bodies is entirely or partially coated by one or more auxiliary agents, wherein the auxiliary agent(s) is/are formed in such a way that it/they reinforces/reinforce the mechanical connection of the active ingredient depot(s) to the implantable body/bodies.

17. Use of one or more active ingredient depots produced in accordance with one of Claims 1 to 7 for producing an active-ingredient-charged, endovascularly implantable body for prophylaxis or treatment of a stenosis, an aneurysm, or a tumour tissue in a human or animal body.

## Revendications

1. Procédé de fabrication d'un dépôt de matière active qui est conçu pour la liaison mécanique avec une surface d'un corps endovasculaire implantable, comprenant ou étant constitué des étapes suivantes :
a) mise à disposition d'un ou de plusieurs polymères,
b) mise à disposition d'une ou de plusieurs matières actives et
c) fabrication d'un dépôt de matière active à partir du ou des polymères et de la ou des matières actives, où le dépôt de matière active est ainsi conçu qu'il peut être lié mécaniquement au moyen de l'effet d'une force avec la surface du corps, de telle manière que le dépôt de matière active se trouve amarré à la surface du corps.

2. Procédé selon la revendication 1, **caractérisé en ce que** le polymère ou l'un des polymères représente des polymères non dégradables ou dégradables et que le ou l'un des polymères est choisi dans le groupe constitué de :
- polymères non dégradables : polyéthylène ; polychlorure de vinyle ; polyacrylate ; de préférence polyéthyl-acrylate et polyméthylacrylate, polyméthylméthacrylate, poly-méthyl-co-éthyl-acrylate et éthylène/éthylacrylate ; polytétrafluroéthylène, de préférence des copolymères éthylène/chlorotrifluoroéthylène, des copolymères éthylène/tétrafluoroéthylène ; polyamide, de préférence de polyimide, PA-11, PA-12, PA-46, PA-66 ; polyétherimide ; polyéthersulfone ; poly(iso)butylène ; chlorure de polyvinyle ; fluorure de polyvinyle ; alcool polyvinylique ; polyuréthane ; polybutylènetéréphtalate ; silicones ; polyphosphazanes ; de mousses de polymères, de préférence des mousses de polymères à base de carbonates, de styrènes ; de copolymères et/ou de mélanges des classes de polymères citées, de polymères de la classe des thermoplastiques ainsi que
- des polymères dégradables : polydioxanone ; polyglycolide ; polycaprolactone ; polylactides, de préférence de poly-L-lactide, poly D,L lactide, et de copolymères ainsi que de mélanges de ceux-ci, dont de préférence le poly(L-lactid-co-glycolide), le poly(D,L-lactid-co-glycolide), le poly(L-lactid-co-D,L-lactide), le poly(L-lactid-co-triméthylène carbonate) ; de copolymères triblocs ; de polysaccharides, de préférence de chitosan, de lévane, d'acide hyaluronique, d'héparine, de dextrane, de cellulose ; de polyhydroxyvalérate ; d'éthylvinylacétate ; d'oxyde de polyéthylène ; de polyphosphorycholine ; de fibrine ; d'albumine ; d'acides polyhydroxy butyriques, de préférence des acides polyhydroxy butyriques atactique, isotactique et/ou syndiotactique, ainsi que leurs mélanges.

3. Procédé selon l'une des revendications précédentes, **caractérisé en ce que** le ou l'un des dépôts de matière active dans l'étape c) est fabriqué sous une forme en tuyau, sous une forme en film, sous une préforme ou sous une forme en filet, la forme en tuyau est fabriquée au moyen d'un procédé par extrusion pure, d'un procédé de soufflage de tuyau ou d'un procédé d'emboutissage et le dépôt de matière active sous forme de film est fabriqué au moyen d'un procédé d'extrusion, de coulage ou de pressage.

4. Procédé selon la revendication 3, comprenant en outre, dans l'étape de procédé c), le fait que le dépôt de matière active sous forme de tuyau est rapporté sur la longueur d'un passage, respectivement d'un support, du stent, la forme en tuyau est fendue le long de l'axe et le tuyau est ainsi transformé qu'il représente un clip avec lequel les passages ou respectivement le support du stent peuvent être reliés mécaniquement au moyen de l'effet d'une force.

5. Procédé selon la revendication 3, **caractérisé en ce que** le dépôt de matière active représente une préforme de corps creux en forme de tuyau, qui est fabriquée au moyen d'un procédé de soufflage d'un corps creux ou d'un procédé de moulage par injection, où la préforme représente le négatif d'un point du stent ou d'un passage, respectivement d'un support du stent sur lequel il est rapporté ou que la préforme est conçue de sorte qu'elle est repliée sur le corps, de préférence le stent, et peut ainsi être reliée mécaniquement avec le corps, de préférence le stent.

6. Procédé selon la revendication 5, **caractérisé en ce que** la préforme se trouve déjà à l'état fendu ou est façonnée dans une autre étape afin qu'elle soit fendue le long de l'axe, et cette préforme est ainsi conçue qu'elle représente un clip qui peut être relié mécaniquement avec le stent au moyen de l'effet d'une force, ou que la préforme est rapportée sur la longueur des passages, respectivement du support, du stent, se présente à l'état fendu ou est fendue le long de l'axe, et cette préforme est ainsi conçue qu'elle représente un clip qui peut être relié mécaniquement avec les passages, respectivement le support des stents au moyen de l'effet d'une force.

7. Procédé selon l'une des revendications précédentes, **caractérisé en ce que** la ou les matières actives sont appropriées pour la prophylaxie ou la thérapie d'une resténose de stent ou d'un traitement de cancer et que la ou les matières actives sont choisies dans le groupe constitué :
de régulateurs de lipides, d'immunosuppresseurs, de vasodilatateurs, de bloqueurs des canaux calciques, d'inhibiteurs calciques, d'antiphlogistiques, d'anti-inflammatoires, d'antiallergiques, d'oligonucléotides, d'oestrogènes, d'angioformateurs, de stéroïdes, de protéines, de peptides, d'inhibiteurs de prolifération, d'analgésiques, d'antirhumatismaux, d'inhibiteurs d'angiogénèse, de cytostatiques.

8. Procédé de fabrication d'un corps endovasculaire implantable chargé de matière active, comprenant ou étant constitué des étapes suivantes :
a) mise à disposition d'un corps endovasculaire implantable,
b) mise à disposition d'une ou de plusieurs matières actives qui sont fabriquées selon l'une des revendications 1 à 7, et
c) liaison mécanique du ou d'un des dépôts de matière active provenant de l'étape b) avec le corps de l'étape a), de telle manière que le dépôt de matière active est amarré à la surface du corps.

9. Procédé selon la revendication 8, comprenant en outre le fait que le ou l'un des corps provenant de l'étape a) est revêtu totalement ou partiellement avec un ou plusieurs produits auxiliaires avant la liaison avec le dépôt de matière active dans l'étape c), où le ou les produits auxiliaires sont ainsi conçus qu'ils renforcent la liaison mécanique du ou des dépôts de matière active avec le ou les corps.

10. Procédé selon les revendications 8 ou 9, **caractérisé en ce que** le ou un des dépôts de matière active sous la forme d'un tuyau est conçu de telle sorte qu'il correspond à la longueur d'un passage, respectivement support, du stent, qu'il est fendu le long de l'axe et représente ainsi un clip qui est relié mécaniquement, sans l'étape de procédé c), avec le passage, respectivement le support, du stent au moyen de l'effet d'une force.

11. Procédé selon les revendications 8 ou 9, **caractérisé en ce que** le ou les dépôts de matière active sont conçus sous forme de films et sont reliés i) avec la surface abluminale du stent, ou ii) avec une partie de celle-ci, ou que la forme en film se présente i) perforée en fonction de la géométrie de la surface abluminale du stent ou ii) perforée complémentairement, où la matériau du film correspond aux domaines du matériau du stent ou que le corps, de préférence le stent, est déroulé avec la surface abluminale sur le film i) ou ii) et est relié mécaniquement au moyen de l'effet d'une force.

12. Procédé selon les revendications 8 ou 9, **caractérisé en ce que** le ou un des dépôts de matière active représente une préforme d'un corps creux en forme de tuyau, qui est fabriqué au moyen d'un procédé de soufflage d'un corps creux ou d'un procédé de moulage par injection, où la préforme représente un endroit négatif du stent ou du passage, respectivement du support, du stent ou que la préforme est ainsi conçue que, dans l'étape c), elle est repliée sur le stent et est ainsi reliée mécaniquement avec le stent.

13. Procédé selon la revendication 12, **caractérisé en ce que** la préforme se présente fendue ou est façonnée dans une autre étape de telle sorte qu'elle soit fendue le long de l'axe, et cette préforme représente un clip qui peut être relié mécaniquement avec le stent au moyen de l'effet d'une force dans l'étape c), ou que la préforme est façonnée dans une autre étape de telle sorte qu'elle soit rapportée sur la longueur d'un passage, respectivement du support, du stent, se présente à l'état fendu ou est fendue le long de l'axe, et cette préforme représente un clip qui est relié mécaniquement avec les passages, respectivement le support, du stent dans l'étape c) au moyen de l'effet d'une force.

14. Corps endovasculaire implantable chargé de matière active, fabriqué d'après un procédé selon l'une des revendications 8 à 13.

15. Ensemble comprenant ou constitué :
a) d'un ou de plusieurs dépôts de matière active fabriqué selon l'une des revendications 1 à 7, et
b) d'un ou de plusieurs corps endovasculaires implantables.

16. Ensemble selon la revendication 15, **caractérisé en ce que** le ou un des corps est revêtu totalement ou partiellement avec un ou plusieurs produits auxiliaires, où le ou les produits auxiliaires sont ainsi conçus qu'ils renforcent la liaison mécanique du ou des dépôts de matière active avec le ou les corps implantables.

17. Utilisation d'un ou de plusieurs dépôts de matière active fabriqués selon l'une des revendications 1 à 7 pour la fabrication d'un corps endovasculaire implantable chargé de matière active pour une prophylaxie ou un traitement d'une sténose, d'un anévrisme ou d'un tissu tumoral dans un corps humain ou animal.
